# EUROPÄISCHE PATENTSCHRIFT

(11) **EP 1 623 224 B1**
(45) Veröffentlichungstag und Bekanntmachung des Hinweises auf die Patenterteilung: **17.09.2008**
(21) Anmeldenummer: 04731887.8
(22) Anmeldetag: 10.05.2004
(51) Int. Cl.: G01N 33/543

(54) **BIOKOMPATIBLE SENSORELEKTRODENANORDNUNG UND VERFAHREN ZU DEREN HERSTELLUNG**
BIOCOMPATIBLE SENSOR ELECTRODE ASSEMBLY AND METHOD FOR THE PRODUCTION THEREOF
ENSEMBLE ELECTRODE DE DETECTION BIOCOMPATIBLE ET SON PROCEDE DE PRODUCTION

(30) Priorität: 09.05.2003 DE 10320898
(43) Veröffentlichungstag der Anmeldung: 08.02.2006
(73) Patentinhaber: Iongate Biosciences GmbH, 65926 Frankfurt am Main (DE)
(72) Erfinder: KELETY, Béla, 65931 Frankfurt am Main (DE); DÖRNER, Wolfgang, 69126 Heidelberg (DE); CZEPAN, Frank, 60385 Frankfurt am Main (DE); KRAUSE, Robin, 65933 Frankfurt am Main (DE)
(74) Vertreter: Hoffmann, Jörg Peter
(86) Internationale Anmeldenummer: PCT/EP2004/004993
(87) Internationale Veröffentlichungsnummer: WO 2004/100229

(56) Entgegenhaltungen:
- EP-A- 1 302 545
- WO-A-01/25775
- WO-A-97/41425
- WO-A-98/23948
- WO-A1-02/074983
- US-A- 5 200 051
- US-A1- 2002 028 478
- SEVIN-LANDAIS A ET AL: "FUNCTIONAL IMMOBILISATION OF THE NICOTINIC ACETYLCHOLINE RECEPTOR IN TETHERED LIPID MEMBRANES" BIOPHYSICAL CHEMISTRY, NORTH-HOLLAND, AMSTERDAM, NL, Bd. 85, Nr. 2/3, 15. Juli 2000 (2000-07-15), Seiten 141-152, XP001083966 ISSN: 0301-4622

## Beschreibung

Die Erfindung betrifft eine biokompatible oder biologisch kompatible Sensorelektrodenanordnung und ein Verfahren zu deren Herstellung.

In vielen Bereichen der chemischen und biochemischen Analysetechnik werden biokompatible oder biologisch kompatible Materialanordnungen verwendet, insbesondere biokompatible oder biologisch kompatible Sensoranordnungen oder Sensorelektrodenanordnungen. Durch diese Materialanordnungen. Sensoranordnungen oder Sensorelektrodenanordnungen werden in der Anwendung bestimmte Messprozesse im Hinblick auf einen chemisch, biologisch oder biochemisch relevanten.Analyten durchgeführt.

Bei Analyseverfahren mit hohen Durchsätzen. z.B. bei so genannten High-Throughput-Screeningverfahren sind verschiedene Charakteristika für die biologisch kompatiblen Materialanordnungen. Sensoranordnungen oder Sensorelektrodenanordnungen wünschenswert, insbesondere im Hinblick auf ihre elektrische Sensitivität, ihre mechanische Stabilität und/oder ihre hohe und kostengünstige Verfügbarkeit. Bei herkömmlichen Materialanordnungen. Sensoranordnungen oder Sensorelektrodenanordnungen werden Trägersubstrate verwendet, die zwar vergleichsweise mechanisch stabil ausgebildet sind, aber ansonsten eine vergleichsweise schwierige Handhabbarkeit mit sich führen und auch nicht unbedingt kostengünstig hergestellt werden können.

Aus der EP 1 302 545 A2 ist ein Enzymbiosensor bekannt, bei welchem auf einem Substrat ein elektrisch leitfähiges Material vorgesehen ist, welches in eine Mehrzahl Elektroden strukturiert ausgebildet ist, wobei zwischen den Elektroden und dem weiteren leitfähigen Material isolierende Lücken vorgesehen sind. Die Strukturierung erfolgt beim Herstellen mittels einer Laserablation oder mittels Strukturierungsverfahren.

Die WO 98/23948 A betrifft Anordnungen unabhängig voneinander adressierbarer und festkörperunterstützter flüssiger Doppelschichtmembranen und deren Herstellung. Bei diesen Anordnungen werden der Oberfläche der Substrate Doppelschichtmembranen ausgebildet, wobei zwischen der Oberfläche des Substrats und der Doppelschichtmembran jeweils ein Flüssigkeitsfilm vorgesehen ist. Eingebettet ist die Struktur jeweils in eine Messlösung.

Aus der US 5.200.051 A sind Biosensoren bekannt, die zur elektrochemischen Detektion von Inhaltsstoffen in Analyten mit biologischer und physiologischer Signifikanz dienen. Dabei wird jeweils eine katalytische Indikatorelektrode aus Metall vorgesehen und von einer kombinierten Bezugs- und Gegenelektrode lateral umgeben. Diese Anordnung wird auf einem Substratwafer aus Silizium aufgebracht und mit einer Deckschicht aus Silan abgedeckt. Im Bereich der Indikatorelektrode wird dann eine so genannte Bioschicht, z.B. eine Enzymschicht, aufgebracht. Als Metalle für die katalytische Indikatorelektrode sind auch Edelmetalle denkbar, Jedoch auch Quecksilber und Kohlenstoff.

Aus der WO 97/41425 A ist eine Biosensoreinrichtung bekannt, mit welcher die Bindung zwischen einem Liganden und einem Rezeptor untersucht werden kann. Dazu wird auf einer leitfähigen Schicht einer Nachweiselektrode eine Monoschicht einer Kohlenwasserstoffkettenverbindung aufgebracht. An die distalen Enden der Kohlenwasserstoffkettenmoleküle sind Liganden ankoppelbar. Diese können dann von Rezeptormolekülen gebunden werden, wobei sich dann über die Nähe zur Nachweiselektrode bei der Bindung ein nachweisbares elektrisches Signal ergibt.

Aus der WO 01/25775 A1 sind Strukturen und Strukturierungsverfahren für Elektroden für elektrochemische Sensorstreifen bekannt, wobei auf einem Basismatertal eines Sensorstreifens ein Elektrodensubstrat vorgesehen wird. Das Elektrodenmaterial wird mit einem nachzuweisenden Reagenz benetzt, wobei dieser Nachweis über eine elektrochemische Reaktion erfolgen kann.

Die US 2002/0028478 A1 betrifft ein Verfahren zum Untersuchen von Biokompartimenten, die in Membranstrukturen eingeschlossen sind. Dabei werden in einer Durchflusszelle auf indirekte Art und Weise Konzentrationen von Inhaltsstoffen gemessen, wobei über einen Feldeffekttransistor, welcher einem zu vermessenden Kulturmedium, ausgesetzt wird, und über die Wasserstoffionenkonzentration im Kulturmedium mittels einer Leitfähigkeitsbestimmung die Konzentration im Kulturmedium abgeleitet wird.

Aus der Publikation "Functional immobilization of the nicotinic acectyl coline receptor ...". Biophys. Chem. (2000) 85. 141-152 ist ein Verfahren zum Immobilisieren nikotinischer Acetylcolinrezeptoren in künstlichen Lipidmembranen bekannt, wobei diese über eine Schwefel-Gold-Bindung mittels langkettiger Stützmoleküle an einer Goldelektrode fixiert sind. Die künstliche Membran ist einem wässrigen Messmedium derart ausgesetzt, dass zwischen der Goldoberfläche und der der Elektrode zugewandten Seite der künstlichen Membran ein wässriges Kompartiment in Kontakt mit dem wässrigen Messmedium vorliegt.

Die WO 02/074983 A1 betrifft eine Sensoranordnung, eine Vorrichtung sowie ein Verfahren zur amperometrischen und/oder potentiometrischen, pharmakologischen Wirkort- und/oder Wirkstofftestung. Es werden mit Protein bestückte Primärträger, z.B. in Form von Vesikeln oder Membranfragmenten, auf eine festkörperunterstützte Membran aufgebracht und in einem wässrigen Kompartiment untersucht. Die festkörperunterstützte Membran besteht aus einer Thiolmonoschicht, welche auf einer Goldelektrode, die ihrerseits auf einem Festkörperträger aufgebracht ist, angeordnet ist und mit einer Lipidmonoschicht abgedeckt wird. Zur Unterstützung des Anhaftens des Goldes am Träger kann eine Haftschicht aus Chrom vorgesehen sein.

Der Erfindung liegt die Aufgabe zugrunde, eine biokompatible oder biologisch kompatible Sensorelektrodenanordnung sowie ein Verfahren zu deren Herstellung anzugeben, bei denen besonders einfach, kostengünstig und gleichwohl zuverlässig handhabbar biokompatible oder biologisch kompatible Materialanordnungen zum Einsatz kommen.

Gelöst wird die Aufgabe bei einer biologisch kompatiblen oder biokompatiblen Sensorelektrodenanordnung erfindungsgemäß mit den Merkmalen des Anspruchs 1. Ferner wird die Aufgabe gelöst bei einem Verfahren zum Herstellen einer biologisch kompatiblen oder biokompatiblen Sensorelektrodenanordnung erfindungsgemäß mit den Merkmalen des Anspruchs 18. Vorteilhafte Weiterbildungen sind jeweils Gegenstand der abhängigen Unteransprüche.

Die erfindungsgemäße biologisch kompatible oder biokompatible Sensorelektrodenanordnung weist mindestens einen Trägersubstratbereich auf, welcher mit einer Oberseite mit einem Oberflächenbereich ausgebildet ist. Des Weiteren ist mindestens eine elektrisch leitfähige Elektrode vorgesehen, welche auf dem Oberflächenbereich des Trägersubstratbereichs oder einem Teil davon ausgebildet sind, insbesondere in strukturierter Art und Weise, und welche mit einer vom Trägersubstratbereich abgewandten Oberseite mit einem Oberflächenbereich ausgebildet sind. Schließlich ist ein Biomaterialbereich vorgesehen, welcher auf dem Oberflächenbereich der Elektrode oder einem Teil davon, insbesondere in strukturierter Art und Weise, mit mindestens einer biokompatiblen oder biologisch kompatiblen Materialkomponente ausgebildet ist. Der Trägersubstratbereich mit der Elektrode darauf oder die Elektrode als solche und/oder jeweils ein Teil davon sind zum Beispiel in Form oder nach Art eines Platinenelements oder einer gedruckten Schaltung ausgebildet.

Alternativ oder zusätzlich ist die Elektrode als eine oder mit einer fotolithografisch prozessierten Struktur oder als ein oder mit einem fotolithografisch prozessierten Element ausgebildet.

Ferner ist alternativ oder zusätzlich die Elektrode als eine oder mit einer aufgeklebt oder auflaminiert prozessierten Struktur oder als ein oder mit einem aufgeklebt oder auflaminiert prozessierten Element vorgesehen.

Ferner alternativ oder zusätzlich ist die Elektrode als eine oder mit einer gedruckt prozessierten Struktur oder als ein oder mit einem gedruckt prozessierten Element ausgebildet.

Des Weiteren ist alternativ oder zusätzlich die Elektrode als eine oder mit einer mikromechanisch und/oder durch Laserablation prozessierten Struktur oder als ein oder mit einem mikromechanisch und/oder durch Laserablation prozessierten Element ausgebildet.

Dies erfolgt jeweils insbesondere direkt auf dem Trägersubstratbereich.

Es ist somit eine Kernidee der vorliegenden Erfindung, die erfindungsgemäße biokompatible Sensorelektrodenanordnung auf der Basis eines Trägersubstratbereichs auszubilden, wobei zwischen dem Trägersubstratbereich und dem Biomaterialbereich mit mindestens einer biokompatiblen Materialkomponente die Elektrode vorgesehen ist.

Eine weitere Kernidee der vorliegenden Erfindung besteht darin, dass der Trägersubstratbereich mit der Elektrode oder dass die Elektrode als solche oder Teile davon als Platinenelement, als gedruckte Schaltung, als fotolithografisch prozessierte Struktur, als aufgeklebte oder auflaminierte Struktur, als mikromechanisch und/oder durch Laserablation prozessierte Struktur und/oder als eine gedruckt prozessierte Struktur vorgesehen sind, insbesondere jeweils auf dem Trägersubstratbereich.

Durch diese erfindungsgemäß vorgesehene Struktur ergeben sich gegenüber dem Stand der Technik dahingehend Vorteile, dass bei der erfindungsgemäßen biokompatiblen Sensorelektrodenanordnung zum Beispiel geringe Material- und Herstellungskosten vorliegen, weil auch mit den vorgeschlagenen Ausbildungsformen und Strukturen im Zusammenhang stehende übliche Massenproduktionstechniken bei der Herstellung verwendet werden können.

Darüber hinaus stellt sich automatisch eine entsprechende mechanische Festigkeit der erfindungsgemäßen biokompatiblen Sensorelektrodenanordnung bei hoher Anwendungszuverlässigkeit und Anwendungsflexibilität ein, wobei auch aufgrund der entsprechenden Prozesstechniken der Aspekt der Miniaturisierung der Strukturen der erfindungsgemäßen biokompatiblen Sensorelektrodenanordnung ausreichend und in zuverlässiger Art und Weise gewürdigt ist.

Zwar kann die Elektrode auch durch epitaktische Aufwachsen, durch Aufdampfen und/oder durch Sputtern und dann besonders gut kontrolliert, wohldefiniert und/oder mit planarer Oberfläche erzeugt sein oder werden. Aber es bieten sich aus Kostengründen und/oder wegen der Vereinfachung unter Umständen gerade Strukturen und Verfahren zu deren Herstellung und entsprechende Techniken an, bei denen eine derartige Kontrolle der Bereichseigenschaften oder Oberflächeneigenschaften des Zwischensubstratbereichs nicht möglich ist, wie z.B. Aufbringen und/oder Strukturieren mittel Fotolithografie, Aufkleben, Auflaminieren, Aufdrucken, Ablation, Laserablation oder dergleichen, falls es auf die Wohldefiniertheit, Kontrollierbarkeit und/oder Planarität der Elektrode oder deren Oberfläche nicht ankommt.

Bei einer bevorzugten Ausführungsform der erfindungsgemäßen biokompatiblen Sensorelektrodenanordnung ist es vorgesehen, dass der Trägersubstratbereich ein chemisch inertes, biologisch inertes und/oder im Wesentlichen elektrisch isolierendes Material aufweist oder aus einem solchen Material gebildet ist. Durch diese Maßnahmen ergeben sich minimale Wechselwirkungen mit der chemischen oder biologischen Umgebung, und es bietet sich gerade die Verwendung als Träger für eine Elektrode an.

Im Hinblick auf die vielfachen Anwendungsmöglichkeiten kann es zusätzlich oder alternativ vorgesehen sein, dass der Trägersubstratbereich ein mechanisch flexibles Material aufweist oder aus einem solchen ausgebildet ist, insbesondere nach Art oder in Form einer Folie. Dies ermöglicht dann die Herstellung von zum Beispiel Einwegsensoren zur einmaligen Verwendung, zum Beispiel für diagnostische oder klinische Zwecke oder für Anwendungen im Feldbetrieb, in situ oder am "Point of Care".

In Bezug auf die Strukturierung der biokompatiblen Materialanordnung als Sensorelektrodenanordnung ist es bei der erfindungsgemäßen Sensorelektrodenanordnung vorgesehen sein, dass für die Elektrode eine metallische Schichtstruktur auf dem Oberflächenbereich des Trägersubstratbereichs ausgebildet ist.

Dabei ist es vorgesehen, dass die Schichtstruktur für die Elektrode mit mindestens einem oder aus mindestens einem zuunterst angeordneten Primärmetallbereich, einer nachfolgenden Hilfsschicht sowie einer zuoberst angeordneten eigentlichen Elektrodenschicht ausgebildet ist.

Die Hilfsschicht dient als Legierungs- und Diffusionsbarriere zwischen dem Primärmetallbereich und der eigentlichen Elektrodenschicht, so dass eine Legierungsbildung aufgrund von Interdiffusion der Materialien des Primärmetallbereichs und der eigentlichen Elektrodenschicht ineinander oder miteinander vermieden wird. Dies erhält zum Beispiel die Spezifizität der eigentlichen Elektrodenschicht, insbesondere dann. wenn es um eine Oberflächenfunktionalisierung oder Oberflächenvergütung der eigentlichen Elektrodenschicht geht.

Der Primärmetallbereich ist mit oder aus Kupfer ausgebildet sein.

Die Strukturierung des Primärmetallbereichs kann in verschiedener Form vorgesehen sein, zum Beispiel in Form eines fotolithografisch strukturierten oder aufgebrachten Primärmetallbereichs. Denkbar sind zusätzlich oder alternativ auch aufgeklebt, auflaminiert, durch Ablation und/oder aufgedruckt prozessierte Primärmetallbereiche.

Die Hilfsschicht ist mit oder aus Nickel ausgebildet.

Es ist vorgesehen, dass die zuoberst angeordnete eigentliche Elektrodenschicht mit oder aus einem Edelmetall ausgebildet ist. Dabei wird vorzugsweise Gold eingesetzt.

Die Hilfsschicht und/oder die zuoberst angeordnete eigentliche Elektrodenschicht können galvanisch ausgebildet sein.

Alternativ oder zusätzlich kann an eine mikromechanische Ausbildung und/oder an eine Laserablation gedacht werden.

Im Hinblick auf die weitere biologische, chemische oder biochemische Kompatibilität der gesamten Sensorelektrodenanordnung kann es vorgesehen sein, dass der Trägersubstratbereich ganz oder teilweise aus einem chemisch inerten, biologisch inerten und/oder gegenüber Proteinen, biologischen und/oder chemischen Wirkstoffen höchstens gering adsorptiven Material ausgebildet ist.

Im Hinblick auf die Materialwahl können beim Trägersubstratbereich ganz unterschiedliche Materialien zum Einsatz kommen:

Zwar ist es denkbar, Glas, Quarz und/oder Glimmer als Materialien für oder im Trägersubstratbereich zu verwenden, weil dann ein besonders wohl definierter, gut kontrollierter und/oder planarer Trägersubstratbereich und/oder Oberflächenbereich vorliegen.

Es ist aber von Vorteil, wenn der Trägersubstratbereich ganz oder teilweise aus PMMA, PEEK, PTFE, POM, FR4, Polyimid, wie z.B. PI oder Kapton, PEN, PET und/oder aus einem - insbesondere im UV-Bereich - transparentem Material ausgebildet ist, falls es auf die Wohldefiniertheit, Kontrolle und/oder Planarität des Trägersubstratbereichs und/oder des Oberflächenbereichs nicht ankommt. Durch diese Materialwahl können der Verarbeitungsaufwand und die Kosten reduziert werden.

Es bedeuten dabei:
- PMMA: Polymethylmethacrylat,
- PEEK: Polyetheretherketon,
- PEN: Polyethylennaphthalat,
- FR4: glasfaserverstärktes Epoxydharz,
- PI: Polyimid,
- PET: Polyethylenterephthalat
- POM: Polyoxymethylen und
- PTFE: Polytetrafluorethylen oder Teflon.

PEEK, POM und PTFE sind in der Regel nicht transparent. PTFE lässt sich gewöhnlich nicht verkleben. Wenn allerdings andere Verbindungstechniken als UV-Verkleben zum Einsatz kommen, können diese Materialien auch sinnvoll verwendet werden.

Im Bezug auf eine möglichst hohe Flächenausbeute und/oder auf eine mögliche Automatisierung ist es ferner von Vorteil, wenn eine Mehrzahl, insbesondere gleichartiger Elektroden und/oder entsprechender Biomaterialbereiche ausgebildet ist. Diese Mehrzahl kann in zusammenhängender oder in getrennter Form ausgebildet sein. Insbesondere bietet sich eine elektrische Isolierung voneinander an, um Beispiel voneinander getrennte und isolierte Sensorelemente der Sensorelektrodenanordnung zu erhalten. Die Mehrzahl Elektroden und/oder Biomaterialbereiche ist zum Beispiel auch in lateral nebeneinander angeordneter Form auf dem Trägersubstratbereich ausgebildet.

Besonders vorteilhaft ist eine Anordnung der Mehrzahl Elektroden und/oder entsprechender Biomaterialbereiche in einer Reihe oder in Matrixform.

Besonders vorteilhaft bietet sich die Anwendung der Sensorelektrodenanordnung gemäß der vorliegenden Erfindung als Sensorelektrodenanordnung zur amperometrischen und/oder potentiometrischen, pharmakologischen Wirkort- und/oder Wirkstofftestung an.

Insbesondere im Hinblick auf eine Anwendung im Bereich der amperometrischen und/oder potentiometrischen, pharmakologischen Wirkort- und/oder Wirkstofftestung können weitere Aspekte wesentlich sein, wie nachfolgend erläutert wird.

Bei der erfindungsgemäßen biokompatiblen oder biologisch kompatiblen Sensorelektrodenanordnung für die amperometrische und/oder potentiometrische, pharmakologische Wirkort- und/oder Wirkstofftestung ist ein elektrisch leitfähiger und festkörperartiger Elektrodenbereich ausgebildet.

Dieser wird zum Beispiel von der Elektrode als Zwischensubstratbereich und insbesondere von der eigentlichen Elektrodenschicht gebildet.

Ferner ist eine Mehrzahl Primärträger vorgesehen, welche in unmittelbarer räumlicher Nachbarschaft des Elektrodenbereichs angeordnet sind und welche zu einer elektrischen Aktion aktivierbare und biologische Einheiten, insbesondere Membranproteine, aufweisen. Darüber hinaus ist ein wässriges Messmedium vorgesehen, in welchem die Primärträger und zumindest ein Teil des Elektrodenbereichs angeordnet sind.

Der Elektrodenbereich ist gegenüber dem Messmedium, den Primärträgern und gegenüber den biologischen Einheiten erfindungsgemäß elektrisch isoliert ausgebildet.

Als Primärträger sind erfindungsgemäß jeweils eine eukariontische Zelle, eine prokariontische Zelle, ein Bakterium, ein Virus oder Bestandteile, insbesondere Membranfragmente, oder Verbände davon in nativer Form oder in abgewandelter Form, insbesondere in gereinigter, mikrobiologisch und/oder molekularbiologisch geänderter Form, vorgesehen. Alternativ oder zusätzlich sind als Primärträger jeweils ein Vesikel, ein Liposom oder eine mizelläre Struktur vorgesehen.

Maßgeblicher Bestandteil der erfindungsgemäßen Sensorelektrodenanordnung ist also ein Elektrodenbereich. Diese Sensorelektrodenanordnung zur amperometrischen und/oder potentiometrischen, pharmakologischen Wirkort- und/oder Wirkstofftestung weist also mindestens einen elektrisch leitfähigen Elektrodenbereich auf. Die Sensorelektrodenanordnung ist ausgebildet, im Betrieb in einem wässrigen Messmedium angeordnet zu werden. Des Weiteren ist die Sensorelektrodenanordnung ausgebildet, eine Mehrzahl oder Vielzahl Primärträger mit zu einer elektrischen Aktion aktivierbaren biologischen Einheiten, insbesondere Membranproteinen oder dergleichen, in unmittelbarer räumlicher Nachbarschaft, insbesondere des Elektrodenbereichs, anzuordnen. Dabei ist zumindest der Elektrodenbereich erfindungsgemäß festkörperartig ausgebildet. Des Weiteren ist dabei erfindungsgemäß der Elektrodenbereich ausgebildet, gegenüber dem vorzusehenden Messmedium und gegenüber den Primärträgern elektrisch isoliert zu sein.

Es ist somit eine weitere Idee der vorliegenden Erfindung, zumindest den Elektrodenbereich der erfindungsgemäßen Sensorelektrodenanordnung festkörperartig oder festkörperunterstützt auszubilden. Dadurch werden der Sensorelektrodenanordnung und insbesondere dem vorgesehenen Elektrodenbereich eine besonders hohe mechanische Stabilität gegeben, wodurch ein besonders robuster und störungsanfälliger Betrieb im Rahmen der Wirkort- und/oder Wirkstofftestung möglich ist.

Erst durch die Festkörperunterstützung wird eine Aktivierung z.B. von Membranproteinen durch einen Konzentrationssprung möglich. Dies kann insbesondere im Rahmen eines schnellen und/oder kontinuierlichen Lösungsaustauschs erfolgen, wodurch - insbesondere bei amperometrischen Messungen - ein hoher Signalpegel und somit eine hohe Empfindlichkeit erreichbar sind. Aufgrund der Robustheit durch die Festkörperunterstützung sind auch eine leichtere Handhabung und ein bequemer Einbau möglich.

Ein weiterer Aspekt der vorliegenden Erfindung besteht darin, den Elektrodenbereich so auszubilden, dass er im Betrieb gegenüber dem Messmedium und gegenüber den Primärträgern elektrisch isoliert ausgebildet ist. Durch diese Maßnahme wird erreicht, dass die Sensorelektrodenanordnung zum Beispiel als kapazitiv gekoppelte Elektrode eingesetzt werden kann. Dies hat insbesondere im Hinblick auf das Signal-zu-Rauschverhältnis, also im Hinblick auf die Nachweisgenauigkeit erhebliche Vorteile. Des Weiteren ist bei der kapazitiven Kopplung der Elektrodenbereich der Sensorelektrodenanordnung an keiner chemischen Umsetzung beteiligt, wie das zum Beispiel bei einer typischen elektrochemischen Halbzelle der Fall wäre.

Als weiterer Kernaspekt der erfindungsgemäßen Sensoranordnung ist die Auswahl der die biologischen Einheiten tragenden Primärträger zu sehen. Die Primärträger können jeweils eukariontische Zellen, prokariontische Zellen, Bakterien, Viren oder Bestandteile, insbesondere Membranfragmente, oder Verbände davon sein, und zwar in nativer Form oder in abgewandelter Form, insbesondere in gereinigter, molekularbiologisch und/oder mikrobiologisch geänderter Form. Alternativ oder zusätzlich sind als Primärträger Vesikel, Liposomen oder mizelläre Strukturen denkbar.

Bei einer besonders vorteilhaften Ausgestaltungsform der erfindungsgemäßen Sensorelektrodenanordnung ist es vorgesehen, dass der Elektrodenbereich mindestens eine elektrische leitfähige Elektrode aufweist, dass ein elektrisch isolierender Isolationsbereich in Form eines Biomaterialbereichs vorgesehen ist und dass durch den Isolationsbereich die jeweilige Elektrode vom Messmedium, von den Primärträgern und von den biologischen Einheiten elektrisch isoliert ist.

Die Elektrode weist eine eigentliche Elektrodenschicht auf. Der Biomaterialbereich bildet dabei den Isolationsbereich.

Der Elektrodenbereich besitzt also vorteilhafterweise mindestens eine Elektrode. Diese kann zum einen jeweils selbst als mechanisch stabiler Materialbereich ausgebildet sein.

Andererseits kann der Elektrodenbereich einen Träger aufweisen, der insbesondere festkörperartig ausgebildet ist. Diese Funktion wird zum Beispiel vom Trägersubstratbereich übernommen. Dann ist es möglich, dass die Elektrode jeweils als Materialbereich oder -schicht auf einem Oberflächenbereich oder der Oberfläche dieses Trägers ausgebildet ist, insbesondere in zusammenhängender Art und Weise. Dabei ist es dann insbesondere vorgesehen, dass die Elektrode durch die Festkörperunterstützung durch den Träger mechanische Stabilität erlangt. Diese Vorgehensweise hat den Vorteil, dass gegebenenfalls hochwertige Materialien zum Beispiel als dünne Schicht auf dem Träger aufgebracht werden können, so dass sich in betriebswirtschaftlicher Hinsicht die Möglichkeit einer Einwegsensorelektrodeneinrichtung bietet, die zu erschwinglichen Preisen herstellbar und auf dem Markt verwertbar ist. Gegebenenfalls kann der Träger, insbesondere der Elektrodenbereich, wieder verwendet werden, wobei insbesondere ein erneuerter Isolationsbereich, z.B. eine neue Thiolschicht, notwendig werden kann.

Vorzugsweise weist die Elektrode mindestens ein metallisches Material auf oder ist aus einem solchen Material gebildet. Dabei wird vorteilhafterweise insbesondere ein chemisch inertes Edelmetall verwendet, vorzugsweise Gold. Denkbar sind insbesondere auch Platin oder Silber.

Ferner ist auch die Verwendung elektrisch leitfähiger Metalloxide für die eigentliche Elekrodenschicht denkbar, z.B. von ITO oder Indium-Zinn-Oxid. Aus oder mit dieser Materialklasse können auch gegebenenfalls vorzusehende Gegenelektroden gefertigt sein oder werden.

Der Träger zur Aufnahme der Elektrode weist also vorteilhafterweise ein elektrisch isolierendes Material auf oder ist aus einem solchen gebildet. Des Weiteren oder alternativ ist es von Vorteil, dass das Material des Trägers im Wesentlichen chemisch inert ist. Vorteilhafterweise bietet sich als Material ein Glas oder dergleichen an. Dabei kann die Form die einer Platte oder dergleichen sein. Die chemische Inertheit verhindert eine Veränderung sowohl des Trägers als auch eine Verunreinigung des Messmediums während des Messprozesses. Durch die Wahl eines elektrisch isolierenden Trägers wird gewährleistet, dass sämtliche Messsignale im Wesentlichen aus dem Bereich der Elektrode stammen.

Eine mögliche Sensorelektrodenanordnung ergibt sich, wenn die Elektrode im Wesentlichen als auf der Oberfläche des Trägers abgeschiedene Materialschicht ausgebildet ist. Es kann sich dabei auch um eine aufgedampfte oder gesputterte Materialschicht handeln. Die Materialschicht zur Ausbildung der Elektrode hat zum Beispiel eine Schichtstärke von etwa 10 nm bis 200 nm.

Zur Ausbildung einer kapazitiven Elektrode und der dazu notwendigen Isolation des Elektrodenbereichs von Messmedium und/oder von den Primärträgern ist vorzugsweise mindestens ein Biomaterialbereich als Isolationsbereich ausgebildet, durch welchen im Betrieb der Elektrodenbereich, insbesondere die Elektrode, im Wesentlichen elektrisch isolierbar ist, insbesondere in Bereichen davon, welche im Betrieb zum mechanischen Kontakt mit dem Messmedium und/ oder mit den Primärträgern vorgesehen sind.

Bei einer weiter bevorzugten Ausführungsform der erfindungsgemäßen Sensorelektrodenanordnung ist der Biomaterialbereich schichtartig ausgebildet. Dabei besteht der Biomaterialbereich zumindest zum Teil aus einer Abfolge von Monoschichten, wobei die Monoschichten als spontan selbstorganisierende Schichten ausgebildet sind.

Dabei ist es von Vorteil, dass als Unterschicht des Biomaterialbereichs oder als unterster oder der Elektrode zugewandter Bereich des Biomaterialbereichs eine Schicht aus einer organischen Thioverbindung vorgesehen ist, im Hinblick auf die elektrischen Eigenschaften und die elektrische Isolation vorzugsweise aus einem langkettigen Alkanthiol, insbesondere aus Oktadekanthiol.

Ferner ist als Oberschicht des Biomaterialbereichs eine Schicht aus einer amphiphilen organischen Verbindung, insbesondere aus einem Lipid, als oberster und von der Elektrode abgewandter Bereich oder Oberflächenbereich des Biomaterialbereichs vorgesehen.

Es kann also von Vorteil sein, den Biomaterialbereich zumindest teilweise schichtartig, insbesondere mehrschichtig, auszubilden. Dadurch werden die Isolationswirkung verstärkt und die Herstellung vereinfacht. Um im Betrieb eine möglichst hohe Rate angelagerter und/oder angeordneter Primärträger im Bereich der Sensorelektrodenanordnung zu erhalten, ist es gemäß einer bevorzugten Ausführungsform der erfindungsgemäßen Sensorelektrodenanordnung vorgesehen, dass zumindest der Oberflächenbereich des Biomaterialbereichs derart abgestimmt ausgebildet ist, dass eine Anlagerung und/oder Anordnung von Primärträgern am Oberflächenbereich des Biomaterialbereichs begünstigt wird, insbesondere in mit der Oberfläche der Primärträger kompatibler Art und Weise. Das bedeutet, dass je nach Oberflächenbeschaffenheit der Primärträger der Oberflächenbereich des Biomaterialbereichs der Sensorelektrodenanordnung entsprechend angepasst ausgebildet ist, so dass sich die Primärträger begünstigt am Oberflächenbereich des Biomaterialbereichs anlagern und dort auch verbleiben.

Im Hinblick auf eine besonders ausgeprägte kapazitive Kopplung der Sensorelektrodenanordnung im Messbetrieb ist es also vorgesehen, dass der Biomaterialbereich zumindest teilweise als Monoschicht, Monolage und/oder als Abfolge davon ausgebildet ist. In diesem Fall ist die auf die Fläche bezogene spezifische elektrische Kapazität der Elektrodengrenzschicht besonders hoch. Besonders einfach gestaltet sich die Anordnung und Ausbildung der erfindungsgemäßen Sensoranordnung, wenn die, Schicht oder die Schichten des Biomaterialbereichs als sich spontan selbstorganisierende Schichten oder als Self-Assembling-Schichten ausgebildet sind oder werden. Dabei werden in vorteilhafter Art und Weise die Neigung und das Bestreben bestimmter, im Wesentlichen flüssiger oder flüssig gelöster Ausgangsstoffe ausgenutzt, auf einer Oberfläche unter Einfluss der Wechselwirkung mit der Struktur der Oberfläche spontan und selbstorganisierend eine geordnete und/oder schichtartige Struktur auszubilden, die unter bestimmten Umständen und bei bestimmten Stoffklassen zur Ausbildung besonders dünner und gegebenenfalls einlagiger Schichten oder Monoschichten, insbesondere von Molekülen, führt.

Beim erfindungsgemäßen Verwenden organischen Thioverbindungen, insbesondere von Alkanthiolen wird ausgenutzt, dass auf bestimmten Edelmetalloberflächen, zum Beispiel Gold, Silber und Platin, aus einer organischen Lösung, welche die entsprechende Thioverbindung in Lösung enthält, aufgrund einer spezifischen chemischen Wechselwirkung der Thiogruppe mit den Oberflächenatomen der Edelmetallelektrode eine kovalent gebundene Monoschicht auf der Elektrodenoberfläche entstehen kann, die bei entsprechender Geometrie der Thioverbindung eine hexagonal dichte Packung auszubilden vermag, wodurch eine besonders geringe Restleitfähigkeit der Edelmetalloberfläche in Bezug auf das vorzusehende Messmedium realisierbar ist.

Entsprechend kann bei der Verwendung von Metalloxiden im Bereich exponierter Oberflächenbereiche der eigentlichen Elektrodenshicht, insbesondere von Indium-Zinn-Oxid, auf eine entsprechend spezifische Siloxanchemie zur Ausbildung einer kovalent gebundenen Unterschicht des Biomaterialbereichs zurückgegriffen werden.

Als Oberschicht oder als oberster und von der Elektrode abgewandter Bereich oder Oberflächenbereich des Biomaterialbereichs kann eine Schicht einer amphiphilen organischen Verbindung vorgesehen sein, insbesondere eines Lipids und/oder dergleichen,

Durch das Vorgehen, bei welchem als Oberschicht oder als oberster und von der Elektrode abgewandter Bereich oder Oberflächenbereich des Biomaterialbereichs eine Schicht einer amphiphilen organischen Verbindung vorgesehen ist oder wird, insbesondere eines Lipids und/oder dergleichen, wird ebenfalls eine besonders wohl definierte Anordnung und Strukturierung der Oberfläche des Biomaterialbereichs erzwungen.

Die amphiphilen organischen Verbindungen besitzen zumindest einen Bereich, der polar ausgebildet ist, so dass sich im Messmedium, welches insbesondere wässrige Natur besitzt, eine gewisse partielle Lösbarkeit ergibt. Andererseits besitzen amphiphile organische Verbindungen einen unpolaren oder hydrophoben Bereich, dessen Anordnung in einem wässrigen Messmedium energetisch weniger bevorzugt ist. Durch diese Phänomene bildet sich bevorzugt eine Schichtstruktur aus, bei welcher die polaren oder wasserlöslichen Bereiche der amphiphilen Verbindung dem wässrigen Messmedium zugeordnet sind, wogegen sich die unpolaren oder hydrophoben Bereiche der amphiphilen organischen Verbindung vom wässrigen Messmedium abgewandt anordnen. Es kann sich somit eine Monoschicht ausbilden, die insbesondere den Oberflächenbereich des Elektrodenbereichs bildet. Dies geschieht bevorzugt in Kombination mit einer Alkanthiolmonoschicht als Unterschicht, so dass sich als Biomaterialbereich zumindest teilweise eine Doppelschicht zweier Monoschichten oder Monolagen ergibt.

Die so ausgebildete Abfolge zweier Monoschichten hat gewisse strukturelle Ähnlichkeiten mit bestimmten Membranstrukturen, die aus biologischen Systemen bekannt sind, so dass man der so ausgebildeten Abfolge zweier Monoschichten - nämlich der der Elektrode zugewandten Alkanthiolmonoschicht und der darüber angeordneten Lipidmonoschicht - eine gewisse Membranstruktur zuordnen kann. Aufgrund des zugrundeliegenden Festkörperträgers bezeichnet man diese Membranstruktur auch als festkörperunterstützte Membran SSM (SSM: solid supported membrane). Diese Membranstruktur SSM hat im Hinblick auf die Anordnung und Eigenschaft der erfindungsgemäßen Sensorelektrodenanordnung als kapazitiv gekoppelte Elektrode besonders günstige Eigenschaften.

Insbesondere weist derjenige Bereich, welcher durch den die Elektrode isolierenden und/oder abdeckenden Bereich des Biomaterialbereichs definiert wird, in vorteilhafter Weise die eben beschriebene Membranstruktur auf. Dabei ist es ferner von Vorteil, dass diese Membranstruktur oder SSM zumindest teilweise eine spezifische elektrische Leitfähigkeit von etwa Gₘ ≈ 1 - 100 nS/cm² besitzt. Des Weiteren liegt in vorteilhafterweise eine spezifische elektrische Kapazität von etwa Cₘ ≈ 10 - 1000 nF/cm² vor. Schließlich ist alternativ oder ergänzend eine Fläche für die Membranstruktur von etwa A ≈ 0,1 - 50 mm² vorgesehen.

Die hohe spezifische Kapazität Cₘ ist von besonderem Vorteil im Hinblick auf eine durchzuführende amperometrische Wirkstofftestung, bei welcher initiierte elektrische Aktionen der im Wesentlichen biologischen Einheiten als elektrische Ströme, nämlich als Verschiebungsströme oder kapazitive Ströme gemessen werden.

Im Hinblick auf das Signalrauschverhältnis ist ein entsprechender Abdichtwiderstand im Bereich von wenigen Nanosiemens von besonderem Vorteil.

Dieser kann gemäß einer anderen Ausführungsform der erfindungsgemäßen Sensoranordnung auch durch Aufbringen einer Teflonschicht, zum Beispiel direkt auf die Metallelektrode, erreicht werden. Ein derartiges Vorgehen ist zum Beispiel für potentiometrische Wirkstofftestungen vollständig ausreichend, da es hier nicht auf eine hohe elektrische Kapazität ankommt, sondern wegen der Spannungsmessung auf den hohen Abdichtwiderstand.

Besonders einfache geometrische Verhältnisse ergeben sich, insbesondere im Hinblick auf die Reproduzierbarkeit der Messergebnisse, wenn der Träger, die Elektrode und/oder der Biomaterialbereich und/oder deren Ober- oder Grenzflächenbereiche zumindest teilweise im Wesentlichen planar ausgebildet sind, insbesondere auch auf mikroskopischer Ebene oder Skala. Die Planarität gewährleistet, dass bestimmte Feldstärkeeffekte an Kanten oder Spitzen, die zum Durchbruch des Abdichtwiderstands führen könnten, ausbleiben. Des Weiteren ergibt sich im Hinblick auf den Austausch des vorzusehenden Messmediums im Betrieb der Vorteil einer homogenen Grenzflächenverteilung. Etwaige Protuberanzen oder Kavitäten würden an der Grenzfläche zwischen dem Biomaterialbereich und dem Messmedium zu Konzentrationsinhomogenitäten führen, die sich unter Umständen nachteilig auf erreichte Nachweis- oder Messergebnisse auswirken könnten. Die Planarität, insbesondere der metallischen Grenzflächen, kann durch entsprechende Herstellungsverfahren, zum Beispiel durch epitaktisches Aufwachsen, Annealen oder dergleichen gewährleistet werden.

Zur externen Kontaktierung der Sensoranordnung, zum Beispiel mit einem äußeren Messkreis oder dergleichen, ist ein Kontaktbereich vorgesehen, wobei insbesondere eine entsprechende Isolation zur Vermeidung sonstiger Kurzschlüsse, insbesondere in Bezug auf das Messmedium, ausgebildet ist.

Besonders vorteilhaft im Hinblick auf einen hohen Durchsatz bei entsprechend durchzuführenden Wirkort- und/oder Wirkstofftests ist es, wenn die erfindungsgemäße Sensoranordnung so ausgebildet ist, dass sie, zumindest im Betrieb, gegenüber Flüssigkeitsströmungen mit hohen Strömungsgeschwindigkeiten, vorzugsweise im Bereich von etwa v ≈ 0.1 - 2 m/s im Wesentlichen konstante mechanische, elektrische und/oder strukturelle Eigenschaften, insbesondere im Bereich der Membranstruktur und/oder insbesondere im Hinblick auf die Anlagerung und/oder Anordnung von Primärträgern, aufweist. Diese geforderte und vorteilhafte Konstanz der mechanischen, elektrischen und/oder strukturellen Eigenschaften der erfindungsgemäßen Sensoranordnung und insbesondere der darin vorgesehenen Membranstruktur ergibt sich bereits inhärent aus den zuvor genannten Maßnahmen zur Ausbildung der Elektrode und der die Elektrode abdeckenden Isolationsschichten, insbesondere in Form von Self-Assembling-Monoschichten aus einem Alkanthiol auf Gold mit einer entsprechenden Monoschicht aus Lipid in einem wässrigen Medium.

Vorteilhafterweise wird die erfindungsgemäße Sensorelektrodenanordnung in einem Verfahren zur amperometrischen und/oder potentiometrischen, pharmakologischen Wirkort- und/oder Wirkstofftestung und in einer Vorrichtung zur Durchführung eines solchen Verfahrens verwendet.

Bei der erfindungsgemäßen Sensorelektrodenanordnung werden als Primärträger Jeweils eine eukariontische Zelle, eine prokariontische Zelle, Organellen davon, eine bakterielle Einheit, eine virale Einheit und/oder dergleichen und/oder Bestandteile, Fragmente, insbesondere Membranfragmente oder dergleichen, und/oder Verbände davon in im Wesentlichen nativer und/oder in abgewandelter, insbesondere gereinigter, mikrobiologisch und/oder molekularbiologisch geänderter. Form vorgesehen.

Es ist somit grundsätzlich denkbar, dass isolierte und ganze Zellen als Primärträger entsprechender biologischer Einheiten, welche zu einer elektrischen Aktion aktivierbar sind, verwendet werden, seien diese pflanzlicher oder tierischer Herkunft. So ist zum Beispiel eine Untersuchung ganzer Herzzellen möglich und denkbar. Andererseits kann auch an die Untersuchung pflanzlicher Zellen, zum Beispiel von Algenzellen oder anderer Einzeller gedacht werden. Darüber hinaus können auch bestimmte Bakterien oder Viren als Ganzes untersucht werden. Ferner ist denkbar, durch bestimmte mikrobiologische oder biochemische Maßnahmen Bestandteile oder Fragmente von Zellen, Bakterien oder Viren, als Primärträger zu verwenden. Weiterhin ist auch denkbar, Verbände von Zellen, Bakterien oder dergleichen als Primärträger zu verwenden und diese an die entsprechende Sensorelektrodeneinrichtung zur Ausbildung einer erfindungsgemäßen Sensoranordnung anzukoppeln.

Des Weiteren besteht erfindungsgemäß die Möglichkeit, sämtliche dieser vorgeschlagenen Primärträger in ihrer nativen Form oder in einer abgewandelten Form zu verwenden. Dabei können zum Beispiel eukariontische Zellen, prokariontische Zellen oder Bakterien verwendet werden, die durch entsprechende Reinigungs-, mikrobiologische und/oder molekularbiologische Verfahren abgeändert wurden, zum Beispiel um bestimmte Proteine mit bestimmten gewünschten Eigenschaften bevorzugt auszubilden.

Neben den in natürlicher Form bereits bereitstehenden Primärträgern in Form von Zellen, Bakterien und dergleichen ist es auch denkbar, künstliche Primärträger zu erzeugen, zum Beispiel in Form von Vesikeln, Liposomen, mizellären Strukturen und/oder dergleichen. Diese werden dann gegebenenfalls mit entsprechenden biologischen Einheiten, welche zu einer elektrischen Aktion aktivierbar sind, versehen und/oder angereichert. Entsprechende Verfahren zur Rekonstitution von Membranproteinen oder dergleichen in Vesikeln oder Liposomen sind bekannt und können hier in vorteilhafter Art und Weise ausgenutzt werden, um besonders vorteilhafte Ausführungsformen der erfindungsgemäßen Sensorelektrodenanordnung zu schaffen.

Als im Wesentlichen biologische Einheiten kommen sämtliche Einheiten in Frage, die zu einer zumindest teilweise elektrisch ausgebildeten Aktion veranlassbar sind. Insbesondere sind derartige biologische Einheiten denkbar, die zumindest zu einem teilweise elektrogenen und/oder elektrophoretischen Ladungsträgertransport und/oder zu einer zumindest teilweise elektrogenen oder elektrophoretischen Ladungsträgerbewegung aktivierbar sind und welche biologische, chemische und biochemische Einheiten darstellen. Dabei handelt es sich insbesondere um Transporteinheiten, die auf ihre Aktivierung hin Ladungsträger bewegen. Denkbar sind auch Bestandteile, Fragmente und/oder Verbände derartiger Einheiten, insbesondere Transporteinheiten.

Es bieten sich insbesondere als biologische Einheiten Membranproteine an, insbesondere Ionenpumpen, Ionenkanäle, Transporter, Rezeptoren und/oder dergleichen. In Bezug auf viele dieser biologischen Einheiten bestehen Erkenntnisse und/oder Vermutungen dahingehend, dass bestimmte Prozesse mit zumindest einem elektrogenen Teilschritt verbunden sind. Diese elektrogenen Teilschritte können mit einem tatsächlichen Stofftransport verbunden sein, wie zum Beispiel bei einem Kanal, einer Ionenpumpe, oder bestimmten Transportern. Es sind aber auch biologische Einheiten, insbesondere Membranproteine, bekannt, deren elektrische Aktivität nicht mit einem Nettostofftransport zusammenhängt, sondern lediglich mit einer, gegebenenfalls reversiblen, Ladungsverschiebung im Rahmen einer Konformationsänderung oder Bindung oder dergleichen. Auch solche elektrischen Aktivitäten sind grundsätzlich als kurzzeitige Verschiebungsströme und/oder Potenzialänderungen erfindungsgemäß messbar.

Die biologischen Einheiten, insbesondere die Membranproteine, können jeweils in im Wesentlichen nativer Form und/oder in abgewandelter, insbesondere gereinigter, mikrobiologisch und/oder molekularbiologisch geänderter Form vorgesehen sein. Zum einen können dadurch bestimmte native Eigenschaften zum Beispiel im Organismus vorhandener Proteine getestet und pharmakologisch untersucht werden. Andererseits bieten sich auch molekularbiologische oder gentechnisch initiierte Veränderungen an, die bestimmten Aspekte, zum Beispiel des Transports oder der pharmakologischen Wirkungsweise eines Wirkstoffes zu analysieren.

Besonders vorteilhaft ist es, dass Primärträger eines jeweils im Wesentlichen einheitlichen Typs von Primärträgern vorgesehen sind. Dies ist im Hinblick auf eine möglichst eindeutige Aussage und Analyse eines Wirkstofftests von Bedeutung und bezieht sich auf die geometrischen, physikalischen, chemischen, biologischen und molekularbiologischen Eigenschaften der Primärträger.

Das Nämliche gilt auch für die in dem Primärträger vorgesehenen biologischen Einheiten, insbesondere für die Membranproteine oder dergleichen. Hier sind biologische Einheiten eines jeweils im Wesentlichen einheitlichen Typs vorgesehen, insbesondere im Hinblick auf ihre geometrischen, physikalischen, chemischen, biologischen und molekularbiologischen Eigenschaften. Zusätzlich sollen die biologischen Einheiten in vorteilhafter Weise im Hinblick auf ihre Orientierung und/oder im Hinblick auf ihre Aktivierbarkeit in Bezug auf den jeweiligen Primärträger in etwa einheitlich sein.

Zur Erzielung einer möglichst hohen Signalqualität ist es von Vorteil, dass die Oberflächen der Primärträger und/oder des Biomaterialbereichs so ausgebildet sind, dass eine Anlagerung und/oder Anordnung der Primärträger am Biomaterialbereich begünstigt wird. Dadurch erhält man zum einen eine besonders hohe Anzahl angelagerter Primärträger und/oder einen besonders innigen Kontakt der Primärträger am Elektrodenbereich, wodurch die elektrische Kopplung und somit das Signal-zu-Rauschverhältnis gesteigert werden.

Die Anlagerung kann z.B. über die so genannte Lipid-Lipid-Wechselwirkung zwischen Primärträger, z.B. Vesikel, und der Biosensorelektrode als Sekundärträger, z.B. Lipid-Thiol-SSM, gesteuert sein. Andererseits ist auch eine kovalente Bindung der Primärträger an der Oberfläche des Biomaterialbereichs denkbar, z.B. in Form eines Biotin-Streptavidin-Schemas oder im Sinne einer His-Tag-Kopplung.

Dabei ist es von besonderem Vorteil, wenn die Oberflächen der Primärträger und des Biomaterialbereichs entgegengesetzt polar zueinander ausgebildet sind. Dies fördert die Anlagerungsrate der Primärträger am Sensor sowie die Stärke des Kontakts zwischen diesen.

Von besonderem Vorteil ist es, wenn als Primärträger im Wesentlichen gleich wirkende und/oder gleichartige Vesikel oder Liposomen, vorzugsweise aus einem Lipid, vorgesehen sind, in und/oder an deren Membran Einheiten im Wesentlichen eines Typs von Membranproteinen in vorzugsweise im Wesentlichen orientierter Form ein- und/oder angelagert sind.

Die erfindungsgemäße Sensorelektrodenanordnung wird vorteilhafterweise von einem Verfahren zur amperometrischen und/oder potentiometrischen, insbesondere pharmakologischen Wirkort- und/oder Wirkstofftestung und/oder bei einer Vorrichtung zur Durchführung eines solchen Verfahrens verwendet.

Gemäß einem weiteren Aspekt der vorliegenden Erfindung wird ein Verfahren zum Herstellen einer biologisch kompatiblen oder biokompatiblen Sensorelektrodenanordnung, insbesondere einer Sensorelektrodenanordnung zur amperometrischen und/oder potentiometrischen, pharmakologischen Wirkort- und/oder Wirkstofftestung gemäß Anspruch 18 geschaffen.

Beim erfindungsgemäßen Herstellungsverfahren wird mindestens ein Trägersubstratbereich mit einer Oberseite mit einem Oberflächenbereich ausgebildet. Ferner wird mindestens eine elektrisch leitfähige Elektrode auf dem Oberflächenbereich des Trägersubstratbereichs oder einem Teil davon, insbesondere in strukturierter Art und Weise, und mit einer vom Trägersubstratbereich abgewandten Oberseite mit einem Oberflächenbereich ausgebildet. Des Weiteren wird ein Biomaterialbereich auf dem Oberflächenbereich der Elektrode oder einem Teil davon, insbesondere in strukturierter Art und Weise, mit mindestens einer biologisch kompatiblen oder biokompatiblen Materialkomponente ausgebildet. Erfindungsgemäß werden der Trägersubstratbereich mit der Elektrode darauf oder die Elektrode als solche und/oder jeweils ein Teil davon in Form oder nach Art eines Platinenelements oder einer gedruckten Schaltung ausgebildet. Alternativ oder zusätzlich ist es vorgesehen, dass der Trägersubstratbereich mit der Elektrode darauf oder die Elektrode als solche und/oder jeweils ein Teil davon als eine oder mit einer fotolithografisch prozessierten Struktur oder als ein oder mit einem fotolithografisch prozessierten Element, als eine oder mit einer aufgeklebt oder auflaminiert prozessierten Struktur oder als ein oder mit einem aufgeklebt oder auflaminiert prozessierten Element, als eine oder mit einer mikromechanisch und/oder durch Laserablation prozessierten Struktur oder als ein oder mit einem mikromechanisch und/oder durch Laserablation prozessierten Element und/oder als eine oder mit einer gedruckt prozessierten Struktur oder als ein oder mit einem gedruckt prozessierten Element ausgebildet werden. Dies ist insbesondere jeweils auf dem Trägersubstratbereich als solchem vorgesehen.

Grundlegende Aspekte des erfindungsgemäßen Herstellungsverfahrens sind also darin zu sehen, dass ein Trägersubstratbereich, eine Elektrode darauf sowie ein Biomaterialbereich auf dem Oberflächenbereich der Elektrode vorgesehen werden. Dabei ist ein weiterer Aspekt, dass der Trägersubstratbereich mit der Elektrode darauf oder die Elektrode als solche und/oder jeweils ein Teil davon in einer für Platinen oder gedruckte Schaltungen möglichen Art und Weise prozessiert werden, um eine besonders zuverlässige und kostengünstige Herstellung, insbesondere in Massenproduktion zu üblichen.

Die weiteren Herstellungsmodi im Hinblick auf das Vorsehen fotolithografisch prozessierter Strukturen oder Elemente, aufgeklebt und/oder auflaminiert prozessierter Strukturen oder Elemente, mikromechanisch und/oder durch Laserablation prozessierter Strukturen oder Elemente und/oder gedruckt prozessierter Strukturen oder Elemente sind zusätzlich oder alternativ vorzusehen.

Die Ablation bzw. Laserablation erfolgt gegebenenfalls durch Masken gestützt.

Entsprechend den oben bereits beschriebenen strukturellen Maßnahmen gestalten sich auch die einzelnen Verfahrensschritte des erfindungsgemäßen Herstellungsverfahrens bzw. deren Abwandlungen.

So ist es gemäß einer bevorzugten Ausführungsform des erfindungsgemäßen Herstellungsverfahrens vorgesehen, dass der Trägersubstratbereich mit einem chemisch inerten, biologisch inerten und/oder im Wesentlichen elektrisch isolierenden Material oder aus einem solchen Material ausgebildet wird.

Weiterhin alternativ oder zusätzlich ist es vorgesehen, dass der Trägersubstratbereich mit einem mechanisch flexiblen Material oder aus einem solchen Material ausgebildet wird, insbesondere in Form oder nach Art einer Folie.

Beim erfindungsgemäßen Herstellungsverfahren ist es vorgesehen, dass für die Elektrode eine metallische Schichtstruktur auf der Oberfläche des Trägersubstratbereichs ausgebildet wird.

Dabei ist es vorgesehen, dass die Schichtstruktur für die Elektrode mit mindestens einem oder aus einem zuunterst angeordneten Primärmetallbereich, einer nachfolgenden Hilfsschicht und, einer zuoberst angeordneten eigentlichen Elektrodenschicht ausgebildet wird.

Der Primärmetallbereich wird mit oder aus Kupfer ausgebildet.

Ferner wird bevorzugt, dass der Primärmetallbereich fotolithografisch ausgebildet wird. Alternativ oder zusätzlich bietet sich ein Prozessieren durch Aufkleben, Auflaminieren, Ablation und/oder Aufdrucken an.

Die Hilfsschicht wird aus Nickel oder Nickel enthaltend gebildet.

Bei einer anderen Ausführungsform der erfindungsgemäßen Herstellungsverfahrens einer Sensorelektrodenanordnung ist es zusätzlich oder alternativ vorgesehen, dass für den die eigentliche Elektrodenschicht der Elektrode eine Schicht aus einem elektrisch leitfähigen Metalloxid, zum Beispiel ITO oder Indium-Zinn-Oxid, ausgebildet wird.

Gemäß einer weiteren alternativen Ausführungsform des erfindungsgemäßen Herstellungsverfahrens wird das zuoberst angeordnete eigentliche Elektrodenmaterial oder die zuoberst angeordnete eigentliche Elektrodenschicht mit oder aus einem Edelmetall ausgebildet, vorzugsweise mit oder aus Gold.

Besonders bevorzugt wird, dass die Hilfsschicht und/oder die zuoberst angeordnete eigentliche Elektrodenschicht galvanisch ausgebildet werden.

Alternativ oder zusätzlich sind ein mikromechanisches Prozessieren und/oder eine Laserablation einsetzbar.

Besonders vorteilhafte Eigenschaften der herzustellenden Sensorelektrodenanordnung ergeben sich dann, wenn gemäß einer bevorzugten Ausführungsform des Herstellungsverfahrens der Trägersubstratbereich ganz oder teilweise aus einem chemisch inerten, biologisch inerten und/oder gegenüber Proteinen, biologischen und/oder chemischen Wirkstoffen höchstens gering absorptiven Material ausgebildet wird.

Bei einer weiteren vorteilhaften Ausgestaltungsform des erfindungsgemäßen Herstellungsverfahrens ist es vorgesehen, dass der Trägersubstratbereich ganz oder teilweise aus PMMA, PTFE, POM, FR4, Polyimid, wie z.B. PI oder Kapton, PEN, PET und/oder aus einem - insbesondere im UV-Bereich - transparentem Material ausgebildet wird.

Zur weiteren Flexibilisierung und Verbreiterung des Einsatzbereiches des herzustellenden Produkts im Sinne der herzustellenden Sensorelektrodenanordnung ist es bei einer weiteren vorteilhaften Weiterbildung des erfindungsgemäßen Herstellungsverfahrens vorgesehen, dass eine Mehrzahl - insbesondere gleichartiger - Elektroden und/oder Biomaterialbereiche ausgebildet wird. Diese können in zusammenhängender oder in getrennter Form ausgebildet werden, insbesondere im Hinblick auf ihre elektrische Verbindung bzw. elektrische Isolation miteinander bzw. voneinander. Dies erfolgt insbesondere in lateral getrennter Art und Weise

In vorteilhafter Weise wird die Mehrzahl Elektroden und/oder Biomaterialbereiche in einer Reihe oder in Matrixform angeordnet.

Gemäß einer weiteren vorteilhaften Ausführungsform des erfindungsgemäßen Herstellungsverfahrens wird die Sensorelektrodenanordnung als Sensorelektrodenanordnung zur amperometrischen und/oder potentiometrischen, pharmakologischen Wirkort- und/oder Wirkstofftestung ausgebildet.

Dabei wird eine Mehrzahl Primärträger in unmittelbar räumlicher Nachbarschaft des Biomaterialbereichs vorgesehen. Die Primärträger enthalten dabei zu einer elektrischen Aktion aktivierbare und biologische Einheiten, insbesondere Membranproteine.

Erfindungsgemäß werden als Primärträger jeweils eine eukaryontische Zelle, eine prokaryontische Zelle, ein Bakterium, ein Virus oder Bestandteile, insbesondere Membranfragmente, oder Verbände davon in nativer Form oder in abgewandelter Form, insbesondere in gereinigter, mikrobiologisch und/oder molekularbiologisch geänderter Form vorgesehen. Alternativ oder zusätzlich werden als Primärträger jeweils ein Vesikel, ein Liposom oder eine zelluläre Struktur vorgesehen.

Die mindestens eine elektrisch leitfähige Elektrode des Elektrodenbereichs wird mit einer Schichtstruktur aus Primärmetallbereich, Hilfsschicht und eigentlicher Elektrodenschicht ausgebildet, wobei der Biomaterialbereich als ein elektrisch isolierender Isolationsbereich vorgesehen wird und wobei durch den Biomaterialbereich die jeweilige Elektrode im Betrieb von einem Messmedium, von den Primärträgern und von den biologischen Einheiten elektrisch isoliert wird.

Beim erfindungsgemäßen Verfahren wird der Biomaterialbereich schichtartig ausgebildet, wobei der Biomaterialbereich zumindest zum Teil aus einer Abfolge von Monoschichten ausgebildet wird und/oder wobei die Monoschichten als spontan selbst organisierende Schichten ausgebildet werden.

Beim erfindungsgemäßen Verfahrens ist es ferner vorgesehen, dass als eine Unterschicht des Biomaterialbereichs eine Schicht aus einem langkettigen Alkanthiol, insbesondere aus Oktadekanthiol, als unterster und der Elektrode zugewandter Bereich und dass als eine Oberschicht des Biomaterialbereichs eine Schicht aus einem Lipid als oberster und von der Elektrode abgewandter Bereich oder Oberflächenbereich des Biomaterialbereichs vorgesehen werden.

Bei einer weiteren vorteilhaften Ausführungsform des erfindungsgemäßen Herstellungsverfahrens ist es vorgesehen, dass der die Elektrode isolierende und abdeckende Bereich des Biomaterialbereichs mit einer Membranstruktur mit einer Fläche von etwa A ≈ 0,1 - 50 mm² und mit einer spezifischen elektrischen Leitfähigkeit von etwa Gₘ ≈ 1 - 100 nS/cm² und/oder mit einer spezifischen Kapazität von etwa Cₘ ≈ 10 - 1000 nF/cm² ausgebildet wird.

Gemäß einer weiteren bevorzugten Ausführungsform des erfindungsgemäßen Herstellungsverfahrens ist es vorgesehen, dass eine biologische Einheit vorgesehen wird, welche zu einer elektrogenen Ladungsträgerbewegung aktivierbar ausgebildet ist, insbesondere zu einem elektrogenen Ladungsträgertransport.

Zusätzlich oder alternativ ist es vorgesehen, dass als biologische Einheit jeweils ein Membranprotein, insbesondere eine Ionenpumpe, ein Ionenkanal, ein Transporter oder ein Rezeptor oder ein Bestandteil oder ein Verband davon vorgesehen wird.

Ferner wird alternativ oder zusätzlich bevorzugt, dass die biologische Einheit in nativer Form oder in abgewandelter Form, insbesondere in gereinigter, mikrobiologisch und/oder molekularbiologisch geänderter Form, vorgesehen wird.

Bei einer weiteren vorteilhaften Fortbildung des erfindungsgemäßen Herstellungsverfahrens ist es vorgesehen, dass die Oberfläche der Primärträger und die Oberfläche des Biomaterialbereichs entgegengesetzt polar oder geladen zueinander ausgebildet werden und/oder dass zwischen der Oberfläche der Primärträger und der Oberfläche des Biomaterialbereichs eine Ankoppelung nach Art einer chemischen Bindung ausgebildet wird, insbesondere über eine His-Tag-Kopplung oder eine Streptavidin-Biotin-Kopplung oder dergleichen.

Diese und weitere Aspekte der vorliegenden Erfindung ergeben sich mit anderen Worten auch aus den nachfolgenden Bemerkungen:

Die Erfindung betrifft neben entsprechenden Strukturen auch ein Verfahren zur Erzeugung elektrisch isolierender, extrem dünner Schichten als biokompatible Bereiche oder Materialbereiche, insbesondere auf Leiterplatten oder dergleichen und deren Verwendung als Sensorelemente, insbesondere zum einmaligen Gebrauch gemäß Anspruch 18.

In der Bioanalytik ist es in bestimmten Fällen wünschenswert, über biokompatible Oberflächen zu verfügen, die zur adsorptiven Anlagerung von biologischen Membranen, Membranfragmenten oder von artifiziellen Lipid-Doppelschichten geeignet sind. Die der hier beschriebenen Erfindung zu Grunde liegende Aufgabe war es, solche Oberflächen möglichst kostengünstig herzustellen, ohne dabei Einschränkungen in der Funktionalität zu erleiden.

Es sind Methoden bekannt, die auf der Grundlage optischer Messgrößen an biokompatiblen Schichten funktionieren, beispielsweise so genannte Biacoremessungen nach dem Prinzip der Oberflächenplasmonenresonanz oder Messungen der Auflaständerung mit der Quarzmikrowaage. In anderen Fällen sollen elektrische Eigenschaften der angelagerten, oft proteinhaltigen Vesikel, Zellen oder Membranfragmente detektiert werden.

Oft können Substrate oder Trägersubstratbereich aus Glimmer, Glas oder Quarz verwendet werden, die dünnschichttechnisch z.B. mit Gold beschichtet sind.

Manchmal ist die Substratwahl aufgrund der spezifischen Substrateigenschaften notwendig, z.B. Glas wegen seiner Transmission im Bereich des sichtbaren Lichtes und wegen seines Brechungsindex, Quarz aufgrund seiner Fähigkeit, im Kondensatorfeld zu Oszillationen anregbar zu sein. Manchmal wird Glas wegen seiner chemischen Inertheit sowie der Möglichkeit zur lithographischen Strukturierung der Goldschicht bis in den Mikrostrukturbereich hinein verwendet.

Glimmer, Glas und/oder Quarz mit der Elektrode zum Beispiel durch epitaktische Aufwachsen, durch Aufdampfen und/oder durch Sputtern zu versehen ist dann sinnvoll und erfindungsgemäß auch vorgesehen, wenn es auf die damit erzielbare Kontrollierbarkeit, Wohldefiniertheit, Hochwertigkeit und/oder Planarität der Elektrode und/oder der entsprechenden Oberflächenbereiche ankommt.

Es ist auch denkbar, dass auf Goldoberflächen Bereiche hergestellt werden können, die zur spezifischen Anbindung von Zielmolekülen dienen können.

Die Herstellung strukturierter biokompatibler Bereiche auf Goldoberflächen, die ihrerseits auf Glassubstrate aufgebracht wurden, ist unter Umständen aufwendig und kostspielig. Glas ist außerdem zerbrechlich und bildet dabei unter Umständen scharfe Kanten, die zu Verletzungen führen können.

Diese Eigenschaften führen dazu, Glassubstrate, Glimmer oder Quarz als Träger für biokompatible Bereiche im Sinn dieser Erfindung erfindungsgemäß durch andere Materialien zu ersetzen und/oder auch andere Beschichtungstechniken als epitaktisches Aufwachsen, Aufdampfen und/oder Sputtern zu verwenden.

Die Verwendung einer selbstorganisierenden Monoschicht, eines Self-assembled Monolayers oder eines SAM führt nicht zu den gewünschten oder auch erforderlichen elektrischen Eigenschaften und nur bedingt zu der Fähigkeit der Oberfläche, Vesikel, Zellen oder Membranfragmente zu adsorbieren.

Eine Kernidee dieser Erfindung ist, zum Beispiel auf in sehr großen Stückzahlen kostengünstig herstellbaren Leiterplatten oder dergleichen biokompatible Bereiche zu erzeugen, die eine sehr geringe elektrische Leitfähigkeit zwischen Elektrode oder eigentlicher Elektrodenschicht und Umgebung vermitteln und geeignete Adsorptionseigenschaften in Bezug auf Zellen, Zellmembranfragmente, Liposomen oder dergleichen aufweisen.

Die Strukturierung der Leiterplatten oder dergleichen erfolgt durch selektives oder strukturiertes Beschichten einer Primärmetallschicht, nämlich einer Kupferschicht, durch nachfolgendes selektives Entfernen der Primärmetallschicht, z.B. nasschemisches Ätzen, und durch anschließendes Veredeln, z.B. durch Vergolden.

Diese Techniken erlauben die Herstellung sehr großer Stückzahlen, wobei die Kosten um ein Vielfaches niedriger sind als die bei der Arbeit auf Glassubstraten. Außerdem können als Biosensor dienende biokompatible Bereiche in unmittelbarer räumlicher Nähe zu Verstärkereinrichtungen auf Platinen hergestellt werden so dass Rauschen und Störeinstrahlung stark reduziert werden können. Die für die Herstellung verwendeten Substrate sind sehr stabil und besitzen gegebenenfalls keine scharfen Kanten. Sie eignen sich daher sehr gut zur Herstellung von Disposables.

Zum Beispiel wird eine mit Kupfer beschichtete Leiterplatte - mit z.B. 17 µm Kupfer - mit Fotolack beschichtet. Ein Layout wird durch Belichtung auf den Fotolack übertragen. Der Fotolack wird entwickelt und an den unbelichteten Stellen spezifisch entfernt, wobei dort die Kupferschicht freigelegt oder exponiert wird. Die Kupferschicht wird an den exponierten Stellen entfernt. Die verbliebenen Lackreste werden ebenfalls entfernt. Auf die freien Kupferstrukturen wird galvanisch Nickel abgeschieden. Auf die so erhaltene Nickelschicht wird galvanisch z.B. Gold abgeschieden.

Auf der Goldschicht wird eine Alkanthiolmonoschicht als Self-Assembled Monolayer oder SAM erzeugt. Auf dem SAM wird durch Zugabe von lipidhaltiger Lösung eine hybride Lipidschicht in Analogie zu einer Lipid-Doppelschicht erzeugt. Diese hybride Lipidschicht ermöglicht die stabile Adsorption z.B. von Membranfragmenten biologischer Membranen, von Zellfragmenten, Vesikeln und Liposomen.

Durch Integration der Leiterplatte oder dergleichen in einen Verstärkerstromkreis und durch Integration des biokompatiblen Bereichs in eine Durchflusszelle und durch Einbringung einer Ag/AgCI-Referenzelektrode in das fluidisch gekoppelte System und durch Anlagerung von Membranfragmenten mit elektrogenen Membranproteinen lassen sich die modifizierten Leiterplatten als Biosensoren verwenden.

Weitere Aspekte der vorliegenden Erfindung ergeben sich, wie folgt:

Man verwendet oft Glas und/oder ein aufwändiges technisches Verfahren, um damit möglichst reproduzierbar dünne, qualitativ sehr hochwertige Elektroden und/oder eigentliche Elektrodenschichten im Sinne der Erfindung, insbesondere Goldschichten zu erhalten. Man ist davon ausgegangen ist, dass eine wenig raue Oberfläche besonders vorteilhaft ist.

Dieses Verfahren ist unter Umständen unwirtschaftlich. Als Alternative können daher Elektroden und/oder eigentliche Elektrodenschichten, also zum Beispiel Goldschichten und entsprechende Oberflächenbereiche von vergleichsweise extrem schlechter Qualität zum Beispiel im Hinblick auf sichtbare Körnigkeit im Lichtmikroskop, Schichtstärke von mehreren Mikrometern, Wulste, Kratzer usw. verwendet werden.

Es zeigt sich jedoch, dass wesentliche Eigenschaften der Membranbiosensorelektrodenbereiche, also der SSM erhalten bleiben, so dass die Materialien Glimmer, Glass, Quarz und/oder die Aufbringung oder Strukturierung mittels epitaktischem Aufwachsen, mittels Aufdampfen und/oder mittels Sputtern nicht notwendig sein müssen.

Also ergeben sich vorteilhafterweise gerade auch Anwendungsgebiete für diese vergleichsweise minderwertigen und aber auch preiswerten Elektroden.

Es ergeben sich erfindungsgemäß somit umfassende Erweiterungen der vergleichsweise aufwendigen Anordnung sowie der entsprechenden Herstellungsverfahren.

Auch deshalb bietet sich die Möglichkeit an, zum Beispiel mit Gold bedampfte Folien, zum Beispiel aus Polyimid oder PEN, mittels Laserablation zu strukturieren, wobei der Laserstrahl durch eine Maske geschickt wird. Die Folie kann von einer Rolle kommen und in einem Endlosprozess strukturiert werden

Nachfolgend wird die vorliegende Erfindung anhand einer schematischen Zeichnung auf der Grundlage bevorzugter Ausführungsbeispiele näher erläutert.
- Fig. 1: zeigt in schematischer und geschnittener Seitenansicht eine Ausführungsform der erfindungsgemäßen biokompatiblen Sensorelektrodenanordnung.
- Fig. 2A, B: zeigen in schematischer Draufsicht bzw. in geschnittener Seitenansicht eine andere Ausführungsform der erfindungsgemäßen biokompatiblen Sensorelektrodenanordnung.
- Fig. 3: zeigt in schematischer Draufsicht eine weitere Ausführungsform der erfindungsgemäßen biokompatiblen Sensorelektrodenanordnung, und zwar mit einer Mehrzahl Einzelelektroden.
- Fig. 4: zeigt eine schematische und teilweise geschnittene Seitenansicht eines anderen Ausführungsbeispiels der erfindungsgemäßen biokompatiblen Sensorelektrodenanordnung mit einem Vesikel als Primärträger und deren Verwendung in einer Messvorrichtung.
- Fig. 5: zeigt eine weitere Ausführungsform der erfindungsgemäßen biokompatiblen Sensorelektrodenanordnung mit einem Membranfragment als Primärträger.

Nachfolgend bezeichnen dieselben Bezugszeichen gleiche, gleichartige oder gleich wirkende Strukturen oder Elemente. Nicht in Jedem Fall ihres Auftretens wird dabei eine detaillierte Beschreibung wiederholt.

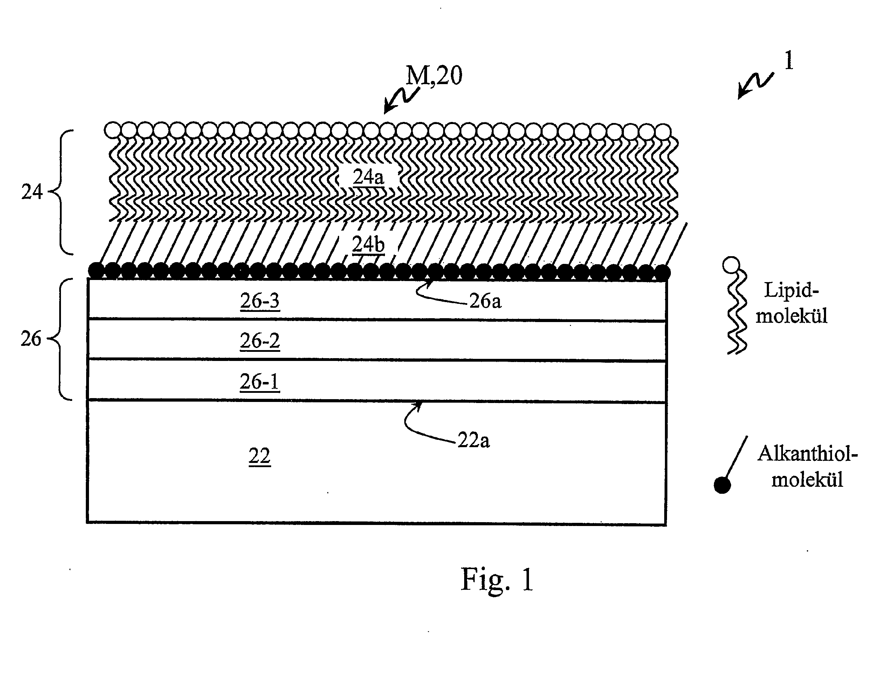

Fig. 1 zeigt in schematischer und geschnittener Seitenansicht eine erste Ausführungsform der erfindungsgemäßen biokompatiblen Sensorelektrodenanordnung 1.

Diese erste Ausführungsform der erfindungsgemäßen biokompatiblen Sensorelektrodenanordnung 1 weist einen Trägersubstratbereich 22 oder eine Trägersubstrat 22 mit einem Oberseitenoberflächenbereich 22a auf, auf welchem die Elektrode 26 als vermittelnder Zwischensubstratbereich in Form einer geschichteten Metallstruktur vorgesehen ist, und zwar mit einem Primärmetallbereich 26-1 aus Kupfer, einer Hilfsschicht 26-2 aus Nickel, die als Diffusionsbarriere und als Legierungsbildungsbarriere dient, sowie einer eigentlichen Elektrodenschicht 26-3, hier aus Gold.

Durch eine spezifische chemische Wechselwirkung mit der eigentlichen Elektrodenschicht 26-3 sind eine Biomaterialschicht 24 oder ein Biomaterialbereich 24 auf dem Oberseitenoberflächenbereich 26a der Elekrode 26 immobilisiert. Diese Biomaterialschicht 24 dient als Isolationsbereich für die erfindungsgemäße Sensorelektrodenanordnung 1 und besteht aus einer geschichteten Abfolge selbstorganisierender Monoschichten 24a und 24b, und zwar aus einer zuunterst angeordneten Unterschicht in Form einer selbstorganisierenden Alkanthiolmonoschicht 24b, welche über die spezifische Thiol-Gold- oder SH-Au-Wechselwirkung angekoppelt ist, und einer zuoberst vorgesehenen selbstorganisierenden Lipidmonoschicht 24a. Durch diese Anordnung wird eine Membranbiosensorelektrodeneinrichtung M, 20 mit einer festkörperunterstützten Membran SSM gebildet.

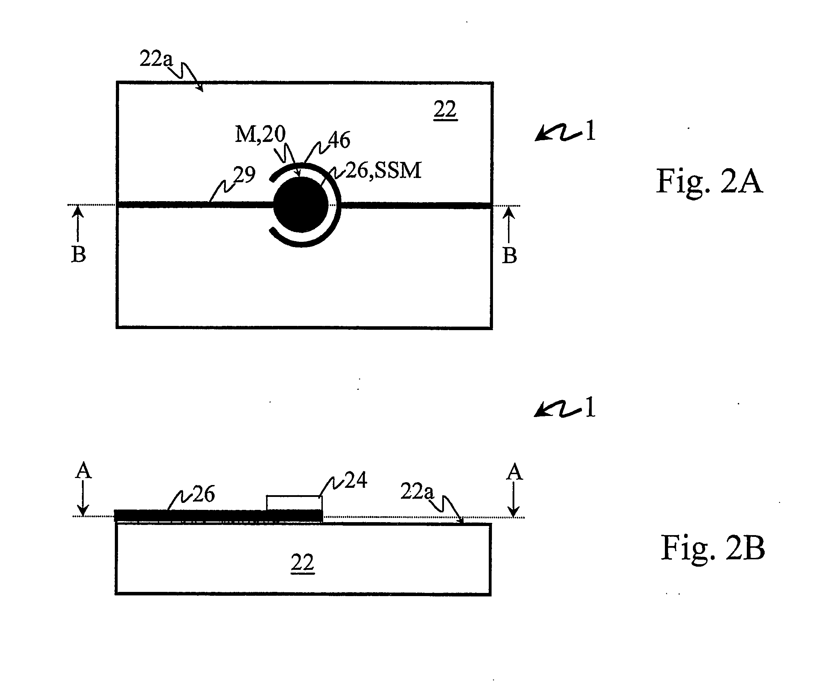

Die Figuren 2A und 2B zeigen in schematischer Draufsicht bzw. in schematischer und geschnittener Seitenansicht eine andere Ausführungsform der erfindungsgemäßen biokompatiblen Sensorelektrodenanordnung 1. Dabei ist in der Draufsicht der Fig. 2A auch ein mit prozessierte Gegenelektrodeneinrichtung 46 dargestellt, die allerdings in der Seitenansicht der Fig. 2B fortgelassen wurde. Diese Gegenelektrode 46 kann auch aus ITO oder Indium-Zinn-Oxid bestehen und alternative Ausgestaltungen annehmen.

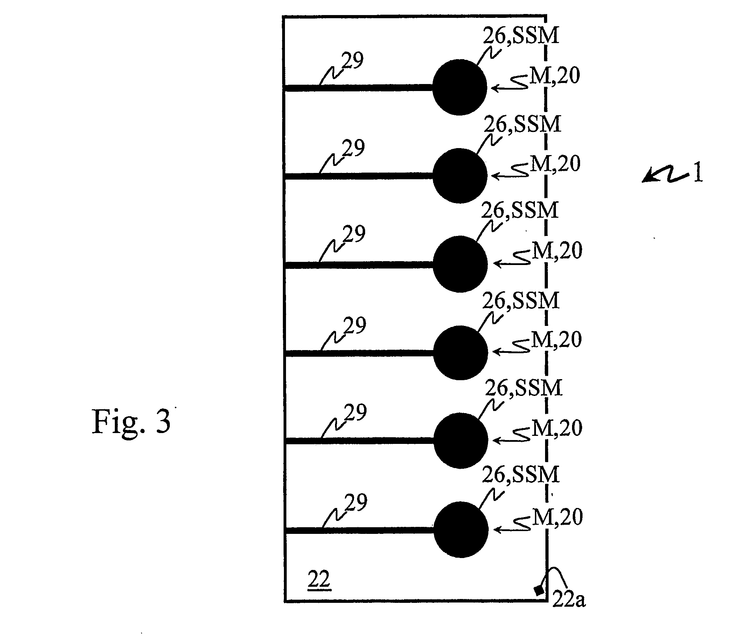

Fig. 3 zeigt mittels einer schematischen Draufsicht eine Ausführungsform der erfindungsgemäßen Sensorelektrodenanordnung 1, auf welcher sechs Einzelelektroden 26 mit entsprechenden Zuleitungen 29 auf der oberen Oberfläche 22a des Trägersubstrats 22 ausgebildet sind. Die einzelnen Elektroden 26 sind mit ihren dazugehörigen Ableitungen 29 im Wesentlichen identisch ausgebildet, zumindest soweit es die Herstellungstoleranzen erlauben.

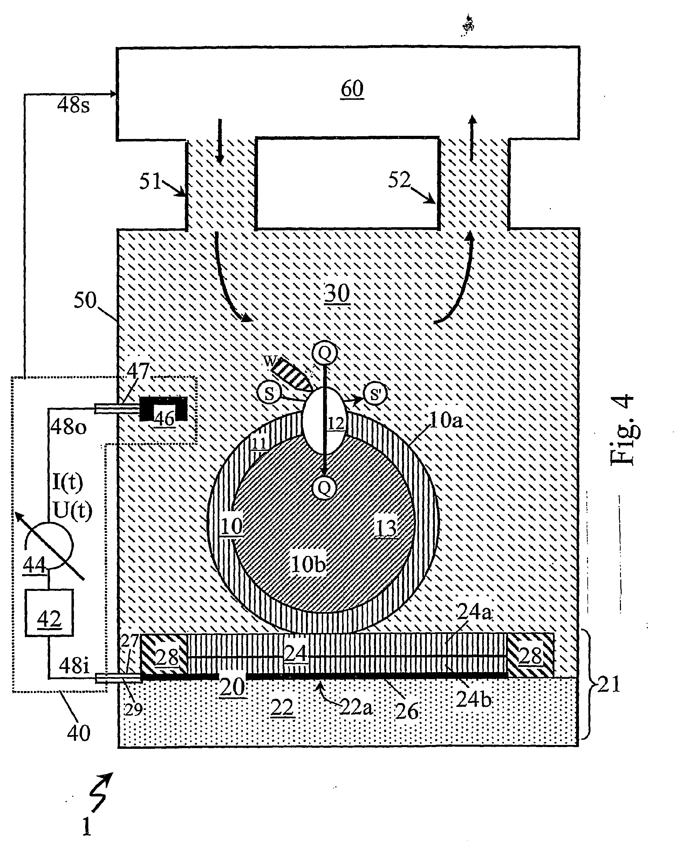

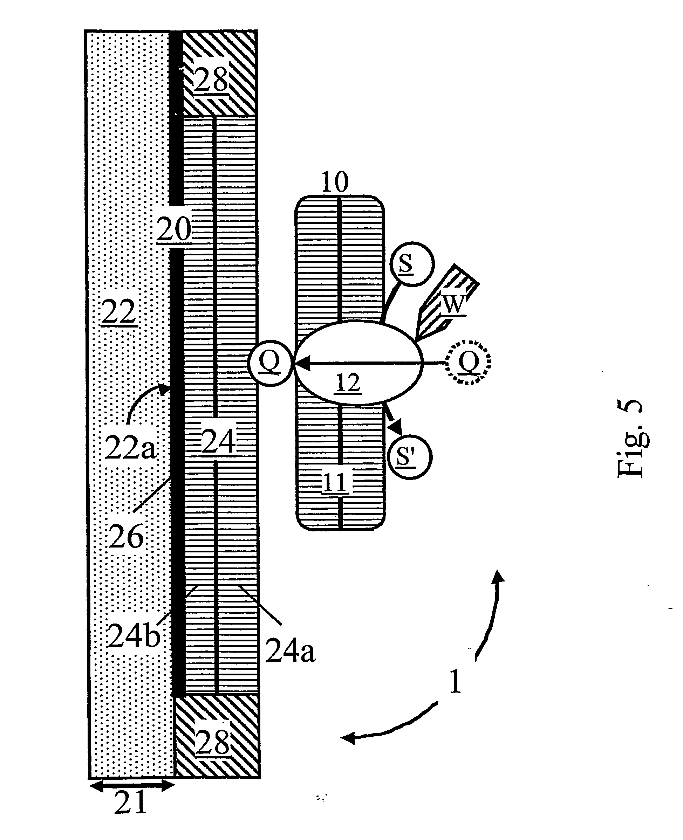

Sämtliche Eigenschaften, welche die mesoskopische oder mikroskopische Struktur der Oberfläche der Membranbiosensorelektrodenbereiche M, der Sensorelektrodenanordnung 20 und insbesondere der jeweiligen zugeordneten Elektroden 26 betreffen, ergeben sich auch aus der Darstellung der nachfolgenden Figuren 4 und 5. Sämtliche dort geschilderten Eigenschaften sind in beliebiger Kombination auf die zuvor in den Fig. 1 bis 3 beschriebenen Strukturen anwendbar. Zusätzlich sei angemerkt, dass die Elektrode 26 dort auch den erfindungsgemäßen Schichtaufbau mit einem Primärmetallbereich 26-1 aus Kupfer, einer Hilfsschicht 26-2 aus Nickel und einer eigentlichen Elektrodenschicht 26-3 aufweisen soll.

Fig. 4 zeigt in einer schematischen und teilweise geschnittenen Seitenansicht eine weitere Ausführungsform der erfindungsgemäßen Sensoranordnung 1 und einer entsprechenden Vorrichtung zur amperometrischen und/oder potentiometrischen pharmakologischen wirkstofftestung.

Ein Messraum 50 in Form eines im Wesentlichen geschlossenen Gefäßes bildet zusammen mit einer Austausch-/Mischeinrichtung 60, zum Beispiel in Form eines Perfusorsystems oder einer Pumpenanlage, einen geschlossenen Flüssigkeitskreislauf. Die Kommunikation der als Messmedium 30 dienenden Flüssigkeit erfolgt über entsprechende Zuführ- und Abführeinrichtungen 51 bzw. 52. Das Messmedium 30 kann dabei eine wässrigere Elektrolytlösung sein, die bestimmte Ionenanteile, eine gegebene Temperatur, einen bestimmten pH-Wert usw. aufweist. Des Weiteren sind im Messmedium 30 gegebenenfalls bestimmte Substratstoffe S und/oder bestimmte Wirkstoffe W enthalten, oder sie werden in späteren Verfahrensschritten durch die Austausch-/Mischeinrichtung 60 hinzugefügt.

Im Messbereich 50 ist eine erfindungsgemäße Sensoranordnung 1 vorgesehen. Die Sensoranordnung 1 besteht aus Primärträgern 10, welche am Oberflächenbereich 24a der als Sekundärträger dienenden Sensorelektrodenanordnung 20 angelagert sind.

In dem in Fig. 4 in schematischer und nicht maßstabsgetreuer Form gezeigten Ausführungsbeispiel ist nur ein einziger Primärträger 10 gezeigt. Dieser besteht aus einem Lipidvesikel oder Liposom in Form einer im Wesentlichen hohlkugelförmig und geschlossen ausgebildeten Lipiddoppelschicht oder Lipidmembran 11. In diese Lipiddoppelschicht 11 des als Primärträger 10 dienenden Vesikels ist als im Wesentlichen biologische Einheit 12 ein Membranprotein membrandurchgreifend eingelagert.

Durch Umsetzung eines im Messmedium 30 vorhandenen Substrats S zu einem umgesetzten Substrat S' werden im Membranprotein 12 bestimmte Prozesse initiiert, die in dem in Fig. 1 gezeigten Fall zu einem Stofftransport einer Spezies Q von der extravesikulären Seite oder Außenseite 10a des Vesikels 10 zur intravesikulären Seite oder Innenseite 10b des Vesikels 10 führt. Ist die Spezies Q mit einer elektrischen Ladung behaftet, so führt der Transport dieser Spezies Q von der Seite 10a zur Seite 10b zu einem Nettoladungstransport, welcher mit einem elektrischen Strom von der Außenseite 10a des Vesikels 10 zur Innenseite 10b des Vesikels 10 korrespondiert.

Zum einen sind in jedem Vesikel 10 in der Regel eine Vielzahl im Wesentlichen identischer Membranproteinmoleküle 12 in im Wesentlichen gleicher Orientierung in der Membran 11 des Vesikels 10 eingebaut. Werden diese im Wesentlichen simultan aktiviert - z.B. durch einen durch Mischen initiierten Konzentrationssprung in der Konzentration des Substrats S von einem nicht aktivierenden Messmedium N, 30 ohne Substrat S zu einem aktivierenden Messmedium A, 30 mit Substrat S - so führt das zu einem messbaren elektrischen Strom.

Dieser Ladungsträgertransport ist deshalb messbar, weil eine Vielzahl von Primärträgern 10 oder Vesikeln an der Oberfläche 24a der Sensorelektrodenanordnung 20 angelagert sind, so dass sich bei Aktivierung einer Vielzahl von Proteinmolekülen 12 in einer Vielzahl von Vesikeln vor der Oberfläche 24a der Sensorelektrodenanordnung 20 eine Raumladung bestimmter Polarität ausbildet. Diese Raumladung wirkt dann auf die Elektrode 26, die in dem in Fig. 1 gezeigten Fall auf einem Träger 22 aus Glas in Form einer Goldschicht aufgedampft ist und durch eine als Isolationsbereich dienende Doppelschicht aus einer unteren Schicht 24b und einer als Oberfläche dienenden Oberschicht 24a abgedeckt und gegenüber dem Messmedium 30 elektrisch isoliert wird.

Die Oberfläche oder obere Schicht 24a des Biomaterialbereichs 24 ist zum Beispiel ein zur Lipiddoppelschicht 11 des Vesikels 10 kompatible Lipidmonoschicht, die mittels eines Self-Assembling-Vorgangs auf einer die untere Schicht 24b bildenden Alkanthiolmonoschicht ausgebildet ist, so dass die Abfolge der Schichten 24b und 24a, nämlich die Abfolge aus einer Alkanthiolmonoschicht und einer Lipidmonoschicht auf einem festkörperartig ausgebildeten Goldsubstrat als Elektrode 26 eine Membranstruktur SSM bildet, die auch als festkörperunterstützte Membran bezeichnet wird (SSM: Solid Supported Membrane).

Über eine Anschlussleitung 48i ist die Sensoranordnung 1 und insbesondere die Sensorelektrodeneinrichtung 20 mit einer Datenerfassungs-/Steuereinrichtung 40 verbunden. Diese weist einen Messeinrichtung 44 auf, in welchem in zeitlicher Abhängigkeit ein elektrischer Strom I(t) oder eine elektrische Spannung U(t) gemessen werden kann. Des Weiteren ist eine Verstärkereinrichtung 42 vorgesehen, in welcher die Messsignale gefiltert und/oder verstärkt werden. Über eine Steuerleitung 48s wird die Wirkstofftestung durch Steuerung der Austausch-/Mischeinrichtung 60 geregelt. Über eine weitere Leitung 48o wird der elektrische Stromkreis mittels einer Gegenelektrode 46, zum Beispiel in Form einer Pt/Pt-Elektrode oder mittels einer Ag/AgCI-Elektrode geschlossen. Isolationen 28, 27 und 47 verhindern Kurzschlüsse der SSM bzw. der Gegenelektrode 46 gegenüber dem Messmedium 30.

Fig. 5 zeigt in schematischer und teilweise geschnittener Seitenansicht eine Ausführungsform der erfindungsgemäßen Sensoranordnung 1, bei welcher als Primärträger 10 anstelle eines Vesikels oder Liposoms ein Membranfragment 10 vorgesehen ist, in welches in orientierter Art und Weise ein als biologische Einheit 12 dienendes Membranprotein eingelagert ist. Auch in Bezug auf die Ausführungsform der Fig. 5 ist festzuhalten, dass die Darstellung nicht maßstabsgetreu ist, und zum anderen in der Regel eine große Mehrzahl von Membranfragmenten gleichzeitig auf der SSM oder der Oberfläche 24a der als Sekundärträger dienenden Sensorelektrodenanordnung 20 angelagert oder adsorbiert sind.

Auch hier ist wieder gezeigt, dass durch Umsetzung des im Messmedium 30 vorgesehenen Substrats S zu einem umgesetzten Substrat S' ein Stofftransport der Spezies Q von einer Seite 10a des Membranfragments 10 zur gegenüberliegenden Seite 10b erfolgt, welcher über den entsprechenden Nettoladungstransport und den damit verbundenen Verschiebungsstrom in zeitlich abhängiger Form nachgewiesen werden kann.

### Bezugszeichenliste

- 1: Sensoranordnung
- 10: Primärträger, Vesikel, Membranfragment
- 10a: Oberfläche, Außenseite, extravesikuläre Seite
- 10b: Innenseite, intravesikuläre Seite
- 11: Membran des Primärträgers 10
- 12: biologische Einheit, Membranprotein
- 13: Innenmedium
- 20: Sensorelektrodenanordnung
- 20a: erste oder obere Oberfläche
- 20c: zweite oder untere Oberfläche
- 21: Elektrodenbereich
- 22: Träger, Trägersubstratbereich
- 22a: Oberflächenbereich, Oberseitenoberflächenbereich
- 24: Biomaterialbereich
- 24a: Oberschicht, Oberflächenbereich, Lipidmonoschicht
- 24b: Unterschicht, Thiol-/Mercaptanmonoschicht
- 26: Elektrode
- 26a: Oberflächenbereich
- 26-1: Primärmetallbereich aus/mit Kupfer
- 26-2: Hilfsschicht mit/aus Nickel als Diffusions-/Legierungsbildungsbarriere
- 26-3: eigentliche Elektrodenschicht
- 27: Isolation
- 28: Isolation
- 29: Anschluss
- 30: Messmedium
- 40: Datenerfassungs-/Steuereinrichtung
- 42: Verstärkereinrichtung
- 44: Messeinrichtung
- 46: Gegenelektrode
- 48i,o: Anschlussleitungen
- 48s: Steuerleitung
- 50: Messbereich, Gefäß, Küvette
- 50a: erste oder obere Oberfläche
- 50c: zweite oder untere Oberfläche
- 51: Zuführeinrichtung
- 51a: Auslassöffnung, Austrittsöffnung
- 52: Abführeinrichtung
- 52e: Einlassöffnung, Eintrittsöffnung
- 53: Vorratsgefäß
- 54: Vorratsgefäß
- 55: Vorratsgefäß
- 60: Austausch-/Mischeinrichtung
- 100: Messvorrichtung

- A: aktivierendes Messmedium
- A+W: aktivierendes Messmedium mit Wirkstoff W
- Cₘ: spezifische Kapazität
- E: Entsorgung
- Gₘ: spezifische Leitfähigkeit
- M: Membranbiosensorelektrodenbereich
- N: nicht aktivierendes Messmedium
- I(t): Stromsignal
- Q: Ladungsträger
- S: Substrat
- S': umgesetztes Substrat
- SSM: Membranstruktur, festkörperunterstützte Membran
- U(t): Spannungssignal
- v: Fließgeschwindigkeit, Strömungsgeschwindigkeit
- W: Wirkstoff
- X: Wirkstoff

## Patentansprüche

1. Biokompatible Sensorelektrodenanordnung (20),
- mit mindestens einem Trägersubstratbereich (22), welcher mit einer Oberseite mit einem Oberflächenbereich (22a) ausgebildet ist,
- mit mindestens einer elektrisch leitfähigen Elektrode (26), welche auf dem Oberflächenbereich (22a) des Trägersubstratbereichs (22) oder einem Teil davon und mit einer vom Trägersubstratbereich (22) abgewandten Oberseite mit einem Oberflächenbereich (26a) ausgebildet ist, und
- mit einem Biomaterialbereich (24), welcher auf dem Oberflächenbereich (26a) der Elektrode (26) oder einem Teil davon mit mindestens einer biologisch kompatiblen Materialkomponente ausgebildet ist,
- wobei der Biomaterialbereich (24) als ein elektrisch isolierender Isolationsbereich vorgesehen ist,
- wobei durch den Biomaterialbereich (24) die Jeweilige Elektrode (26) im Betrieb von einem Messmedium (30), von den Primärträgern (10) und von den biologischen Einheiten (12) elektrisch isoliert ist,
- wobei der Biomaterialbereich (24) schichtartig ausgebildet ist.
- wobei der Biomaterialbereich (24) zumindest zum Teil aus einer Abfolge von Monoschichten (24a. 24b) ausgebildet ist,
- wobei die Monoschichten (24a, 24b) als spontan selbstorganisierende Schichten ausgebildet sind,
- wobei als eine Unterschicht (24b) des Biomaterialbereichs (24) eine Schicht aus einem langkettigen Alkanthiol als unterster und der Elektrode (26) zugewandter Bereich (24b) des Biomaterialbereichs (24) ausgebildet ist,
- wobei als eine Oberschicht (24a) des Biomaterialbereichs (24) eine Schicht aus einem Lipid als oberster und von der Elektrode (26) abgewandter Bereich oder Oberflächenbereich des Biomaterialbereichs (24) ausgebildet ist,
- wobei der Trägersubstratbereich (22) mit der Elektrode (26) darauf als solche oder die Elektrode (26) als solche einerseits und der Biomaterialbereich (24) andererseits in strukturierter Art und Weise, nämlich in Form oder nach Art eines Platinenelements oder einer gedruckten Schaltung ausgebildet sind,
- wobei für die Elektrode (26) eine metallische Schichtstruktur auf der Oberfläche (22a) des Trägersubstratbereich (22) ausgebildet ist,
- wobei die Schichtstruktur für die Elektrode (26) mit mindestens einem zuunterst angeordneten Primärmetallbereich (26-1), einer nachfolgenden Hilfsschicht (26-2) als Legierungs- und Diffusionsbarriere und einer zuoberst angeordneten eigentlichen Elektrodenschicht (26-3) ausgebildet ist,
- wobei der Primärmetallbereich (26-1) mit oder aus Kupfer ausgebildet ist,
- wobei Hilfsschicht (26-2) mit oder aus Nickel ausgebildet ist und
- wobei die zuoberst angeordnete eigentliche Elektrodenschicht (26-3) mit oder aus einem Edelmetall ausgebildet ist

2. Sensorelektrodenanordnung nach Anspruch 1,
**dadurch gekennzeichnet.**
**dass** der Trägersubstratbereich (22) ein chemisch inertes und elektrisch isolierendes Material aufweist oder aus einem solchen gebildet ist.

3. Sensorelektrodenanordnung nach einem der vorangehenden Ansprüche,
**dadurch gekennzeichnet,**
**dass** der Trägersubstratbereich (22) ein mechanisch flexibles Material aufweist oder als ein solches ausgebildet ist, insbesondere in Form oder nach Art einer Folie.

4. Sensorelektrodenanordnung nach einem der vorangehenden Ansprüche,
**dadurch gekennzeichnet,**
**dass** der Primärmetallbereich (26-1) fotolithografisch, aufgeklebt, auflaminiert und/oder aufgedruckt prozessiert ausgebildet ist.

5. Sensorelektrodenanordnung nach einem der vorangehenden Ansprüche,
**dadurch gekennzeichnet,**
**dass** die Hilfsschicht (26-2), die zuoberst angeordnete eigentliche Elektrodenschicht (26-3) oder beide galvanisch ausgebildet sind.

6. Sensorelektrodenanordnung nach einem der vorangehenden Ansprüche,
**dadurch gekennzeichnet.**
**dass** der Trägersubstratbereich (22) ganz oder teilweise aus einem chemisch inerten und gegenüber Proteinen, biologischen und chemischen Wirkstoffen höchstens gering adsorptiven Material ausgebildet ist.

7. Sensorelektrodenanordnung nach einem der vorangehenden Ansprüche,
**dadurch gekennzeichnet,**
**dass** der Trägersubstratbereich (22) mit oder aus einem Material aus der Gruppe ausgebildet ist, die besteht aus PMMA, PTFE, POM, FR4, Polyimid, PI, Kapton, PEN, PET und im UV-Bereich transparenten Materialien.

8. Sensorelektrodenanordnung nach einem der vorangehenden Ansprüche,
**dadurch gekennzeichnet,**
**dass** eine Mehrzahl gleichartiger Elektroden (26) und Biomaterialbereiche (24) ausgebildet ist in elektrisch voneinander isolierter Form und lateral nebeneinander auf dem Trägersubstratbereich (22).

9. Sensorelektrodenanordnung nach Anspruch 8,
**dadurch gekennzeichnet,**
**dass** die Mehrzahl Elektroden (26) und Biomaterialbereiche (24) in einer Reihe oder in Matrixform angeordnet sind.

10. Sensorelektrodenanordnung nach einem der vorangehenden Ansprüche, welche zur amperometrischen und/oder potentiometrischen, pharmakologischen Wirkort- und/oder Wirkstofftestung mit einer Gegenelektrode (46) ausgebildet ist.

11. Sensorelektrodenanordnung nach einem der vorangehenden Ansprüche,
bei welcher eine Mehrzahl Primärträger (10) am Biomaterialbereich (24) adsorbiert vorgesehen ist, wobei die Primärträger zu einer elektrischen Aktion aktivierbare und biologische Einheiten (12), insbesondere Membranproteine, enthalten.

12. Sensorelektrodenanordnung nach Anspruch 11.
- bei welcher als Primärträger (10) jeweils ein Primärträger aus der Gruppe vorgesehen ist, die besteht aus einer eukariontischen Zelle, einer prokariontischen Zelle, einem Bakterium, einem Virus, Bestandteilen, Membranfragmenten, oder Verbänden davon in nativer Form oder in abgewandelter Form insbesondere in gereinigter, mikrobiologisch, molekularbiologisch geänderter Form, oder
- bei welcher als Primärträger (10) ein Primärträger aus der Gruppe vorgesehen ist, die besteht aus einem Vesikel, einem Liposom und einer mizellären Struktur.

13. Sensorelektrodenanordnung nach einem der vorangehenden Ansprüche,
**dadurch gekennzeichnet,**
**dass** der die Elektrode (26) isolierende und abdeckende Bereich des Biomaterialbereichs (24) oder des Isolationsbereichs (24) mit einer Membranstruktur (SSM) mit einer Fläche von etwa A ≈ 0.1 - 50 mm² und mit einer spezifischen elektrischen Leitfähigkeit von etwa Gₘ ≈ 1 - 100 nS/cm² und/oder mit einer spezifischen Kapazität von etwa Cₘ ≈ 10 - 1000 nF/cm² ausgebildet ist.

14. Sensorelektrodenanordnung nach einem der vorangehenden Ansprüche,
**dadurch gekennzeichnet,**
**dass** eine biologische Einheit (12) vorgesehen ist, welche zu einer elektrogenen Ladungsträgerbewegung oder zu einem elektrogenen Ladungsträgertransport aktivierbar ausgebildet ist.

15. Sensorelektrodenanordnung nach einem der vorangehenden Ansprüche,
**dadurch gekennzeichnet,**
**dass** als biologische Einheit (12) jeweils ein Membranprotein, eine Ionenpumpe, ein Ionenkanal, ein Transporter, ein Rezeptor, ein Bestandteil oder ein Verband davon vorgesehen ist.

16. Sensorelektrodenanordnung nach einem der vorangehenden Ansprüche,
**dadurch gekennzeichnet,**
**dass** die biologische Einheit (12) in nativer Form oder in abgewandelter, gereinigter, mikrobiologisch oder molekularbiologisch geänderter Form vorgesehen ist.

17. Sensorelektrodenanordnung nach einem der vorangehenden Ansprüche,
**dadurch gekennzeichnet.**
- **dass** die Oberfläche (10a) der Primärträger (10) und die Oberfläche des Biomaterialbereichs (24) entgegengesetzt polar oder geladen zueinander ausgebildet sind oder
- **dass** zwischen der Oberfläche (10a) der Primärträger (10) und der Oberfläche des Biomaterialbereichs (24) eine Ankopplung nach Art einer chemischen Bindung ausgebildet ist über eine His-Tag-Kopplung oder über eine Streptavidin-Biotin-Kopplung.

18. Verfahren zum Herstellen einer biokompatiblen Sensorelektrodenanordnung (20),
- bei welchem mindestens ein Trägersubstratbereich (22) mit einer Oberseite mit einem Oberflächenbereich (22a) ausgebildet wird,
- bei welchem mindestens eine elektrisch leitfähige Elektrode (26) auf dem Oberflächenbereich (22a) des Trägersubstratbereichs (22) oder auf einem Teil davon mit einer vom Trägersubstratbereich (22) abgewandten Oberseite mit einem Oberflächenbereich (26a) ausgebildet wird und
- bei welchem ein Biomaterialbereich (24) auf dem Oberflächenbereich (26a) der Elektrode (26) oder einem Teil davon mit mindestens einer biologisch kompatiblen Materialkomponente ausgebildet wird,
- wobei der Biomaterialbereich (24) als ein elektrisch isolierender Isolationsbereich (24) vorgesehen wird,
- wobei durch den Biomaterialbereich (24) die jeweilige Elektrode (26) im Betrieb von einem Messmedium (30), von den Primärträgern (10) und von den biologischen Einheiten (12) elektrisch isoliert wird,
- wobei der Biomaterialbereich (24) schichtartig ausgebildet wird,
- wobei der Biomaterialbereich (24) zumindest zum Teil aus einer Abfolge von Monoschichten (24a. 24b) ausgebildet wird,
- wobei die Monoschichten (24a. 24b) als spontan selbstorganisierende Schichten ausgebildet werden,
- wobei als eine Unterschicht (24b) des Biomaterialbereichs (24) eine Schicht aus einem langkettigen Alkanthiol als unterster und der Elektrode (26) zugewandter Bereich (24b) des Biomaterialbereichs (24) ausgebildet wird,
- wobei als eine Oberschicht (24a) des Biomaterialbereichs (24) eine Schicht aus einem Lipid als oberster und von der Elektrode (26) abgewandter Bereich oder Oberflächenbereich (24a) des Biomaterialbereichs (24) ausgebildet wird,
- wobei der Trägersubstratbereich (22) mit der Elektrode (26) darauf als solche oder die Elektrode (26) als solche einerseits und der Biomaterialbereich (24) andererseits in strukturierter Art und Weise, nämlich in Form oder nach Art eines Platinenelements oder einer gedruckten Schaltung ausgebildet werden.
- wobei für die Elektrode (26) eine metallische Schichtstruktur auf der Oberseitenoberfläche (22a) des Trägersubstratbereich (22) ausgebildet wird,
- wobei die Schichtstruktur für die Elektrode (26) mit mindestens einem oder aus mindestens einem zuunterst angeordneten Primärmetallbereich (26-1), einer nachfolgenden Hilfsschicht (26-2) als Legierungs- und/oder Diffusionsbarriere (26-2) und einer zuoberst angeordneten eigentlichen Elektrodenschicht (26-3) ausgebildet wird.
- wobei der Primärmetallbereich (26-1) mit oder aus Kupfer ausgebildet wird.
- wobei die Hilfsschicht (26-2) mit oder aus Nickel ausgebildet wird, und
- die zuoberst angeordneten eigentlichen Elektrodenschicht (26-3) mit oder aus einem Edelmetall ausgebildet wird

19. Verfahren nach Anspruch 18.
bei welchem der Trägersubstratbereich (22) mit der Elektrode (26) darauf als solche oder die Elektrode (26) als solche oder jeweils ein Teil davon gebildet werden:
- als eine oder mit einer fotolithografisch prozessierten Struktur oder als ein oder mit einem fotolithografisch prozessierten Element,
- als eine oder mit einer aufgeklebt oder auflaminiert prozessierten Struktur oder als ein oder mit einem aufgeklebt oder auflaminiert prozessierten Element,
- als eine oder mit einer mikromechanisch und/oder durch Laserablation prozessierten Struktur oder als ein oder mit einem mikromechanisch und/oder durch Laserablation prozessierten Element und/oder
- als eine oder mit einer gedruckt prozessierten Struktur oder als ein oder mit einem gedruckt prozessierten Element.

20. Verfahren nach einem der vorangehenden Ansprüche 18 oder 19,
**dadurch gekennzeichnet,**
**dass** der Trägersubstratbereich (22) mit einem chemisch inerten und elektrisch isolierenden Material oder aus einem solchen Material ausgebildet wird.

21. Verfahren nach einem der vorangehenden Ansprüche 18 bis 20,
**dadurch gekennzeichnet,**
**dass** der Trägersubstratbereich (22) mit einem mechanisch flexiblen Material oder aus einem solchen Material ausgebildet wird, insbesondere in Form oder nach Art einer Folie.

22. Verfahren nach einem der vorangehenden Ansprüche 18 bis 21,
**dadurch gekennzeichnet,**
**dass** der Primärmetallbereich (26-1) fotolithografisch, aufgeklebt, auflaminiert oder aufgedruckt prozessiert ausgebildet wird.

23. Verfahren nach einem der vorangehenden Ansprüche 18 bis 22,
**dadurch gekennzeichnet,**
**dass** die Hilfsschicht (26-2), die zuoberst angeordneten eigentlichen Elektrodenschicht (26-3) oder beide galvanisch ausgebildet werden.

24. Verfahren nach einem der vorangehenden Ansprüche 18 bis 23,
**dadurch gekennzeichnet,**
**dass** der Trägersubstratbereich (22) ganz oder teilweise aus einem chemisch inerten und/oder gegenüber Proteinen, biologischen und/oder chemischen Wirkstoffen höchstens gering adsorptiven Material ausgebildet wird.

25. Verfahren nach einem der vorangehenden Ansprüche 18 bis 24,
**dadurch gekennzeichnet,**
**dass** der Trägersubstratbereich (22) ganz oder teilweise aus PMMA, PTFE, POM, FR4, Polyimid, wie z.B. PI oder Kapton, PEN, PET und/oder aus einem - insbesondere im UV-Bereich - transparentem Material ausgebildet wird.

26. Verfahren nach einem der vorangehenden Ansprüche 18 bis 35,
**dadurch gekennzeichnet,**
**dass** eine Mehrzahl gleichartiger Elektroden (26) und Biomaterialbereiche (24) in zusammenhängender oder in getrennter Form ausgebildet wird, in elektrisch voneinander isolierter Form.

27. Verfahren nach Anspruch 26,
**dadurch gekennzeichnet,**
**dass** die Mehrzahl Elektroden (26) und Biomaterialbereiche (24) in einer Reihe oder in Matrixform angeordnet werden.

28. Verfahren nach einem der vorangehenden Ansprüche 18 bis 27,
bei welchem die Sensorelektrodenanordnung zur amperometrischen und/oder potentiometrischen, pharmakologischen Wirkort- und/oder Wirkstofftestung mit einer Gegenelektrode (46) ausgebildet wird.

29. Verfahren nach einem der vorangegangenen Ansprüche 18 bis 28,
bei welchem eine Mehrzahl Primärträger (10) am Biomaterial (24) adsorbiert vorgesehen wird, wobei die Primärträger zu einer elektrischen Aktion aktivierbare und biologische Einheiten (12), insbesondere Membranproteine, enthalten.

30. Verfahren nach Anspruch 29,
- bei welchem als Primärträger (10) jeweils ein Primärträger aus der Gruppe vorgesehen wird, die aufweist eine eukariontische Zelle, eine prokariontische Zelle, ein Bakterium, ein Virus oder Bestandteile, Membranfragmente oder Verbände davon in nativer Form oder in abgewandelter, gereinigter, mikrobiologisch oder molekularbiologisch geänderter Form, oder
- bei welchem als Primärträger (10) jeweils ein Primärträger aus der Gruppe vorgesehen wird, die aufweist ein Vesikel, ein Liposom oder eine mizelläre Struktur vorgesehen wird.

31. Verfahren nach einem der vorangehenden Ansprüche 18 bis 30,
**dadurch gekennzeichnet,**
**dass** der die Elektrode (26) isolierende und abdeckende Bereich des Biomaterialbereichs (24) mit einer Membranstruktur (SSM) mit einer Fläche von etwa A ≈ 0.1 - 50 mm² und mit einer spezifischen elektrischen Leitfähigkeit von etwa Gₘ ≈ 1 - 100 nS/cm² und/oder mit einer spezifischen Kapazität von etwa Cₘ ≈ 10 - 1000 nF/cm² ausgebildet wird.

32. Verfahren nach einem der vorangehenden Ansprüche 18 bis 31
**dadurch gekennzeichnet,**
**dass** eine biologische Einheit (12) vorgesehen wird, welche zu einer elektrogenen Ladungsträgerbewegung aktivierbar ausgebildet wird, insbesondere zu einem elektrogenen Ladungsträgertransport.

33. Verfahren nach einem der vorangehenden Ansprüche 18 bis 32,
**dadurch gekennzeichnet,**
**dass** als biologische Einheit (12) jeweils ein Membranprotein, insbesondere eine Ionenpumpe, ein Ionenkanal, ein Transporter oder ein Rezeptor oder ein Bestandteil oder ein Verband davon ausgebildet wird.

34. Verfahren nach einem der vorangehenden Ansprüche 18 bis 33,
**dadurch gekennzeichnet,**
**dass** die biologische Einheit (12) in nativer Form oder in abgewandelter Form, insbesondere gereinigter, mikrobiologisch und/oder molekularbiologisch geänderter Form, ausgebildet wird.

35. Verfahren nach einem der vorangehenden Ansprüche 18 bis 34
**dadurch gekennzeichnet,**
- **dass** die Oberfläche (10a) der Primärträger (10) und die Oberfläche des Biomaterialbereichs (24) entgegengesetzt polar oder geladen zueinander ausgebildet werden und/oder
- **dass** zwischen der Oberfläche (10a) der Primärträger (10) und der Oberfläche des Biomaterialbereichs (24) eine Ankopplung nach Art einer chemischen Bindung ausgebildet wird, insbesondere über eine His-Tag-Kopplung oder eine Streptavidin-Biotin-Kopplung.

## Claims

1. Biocompatible sensor electrode arrangement (20), with:
- at least one carrier substrate area (22) which is formed with a top side with a surface area (22a),
- at least one electrical conductive electrode (26) which is formed on the surface area (22a) of the carrier substrate area (22) or a part thereof and which is formed with a top side facing away from the carrier substrate area (22) with a surface area (26a) and
- a biomaterial area (24) which is formed on the surface area (26a) of the electrode (26) or a part thereof with at least one biologically compatible material component,
- the biomaterial area (24) being provided as an electrically insulating insulation area and
- in operation, the electrode (26) concerned being electrically insulated by the biomaterial area (24) from a measuring medium (30), from the primary carriers (10) and from the biological units (12),
- the biomaterial area (24) being formed as layers,
- the biomaterial area (24) being formed at least partly of a sequence of monolayers (24a, 24b),
- the monolayers (24a), (24b) being formed as spontaneously self-organising layers,
- as sub-layer (24b) of the biomaterial area (24), a layer of a long-chain alkane thiol being provided as bottom most area (24b) facing towards the electrode (26) of the biomaterial area (24),
- as top layer (24a) of the biomaterial area (24), a layer of a lipid being provided as uppermost area facing away from the electrode (26) or surface area of the biomaterial area (24),
- the carrier substrate area (22) with the electrode (26) thereon as such or the electrode (26) as such on the one hand and the biomaterial area (24) on the other hand are formed in a structured manner, namely in the form or the manner of a wafer element or a printed circuit,
- wherein for the electrode (26), a metallic layer structure is formed on the surface (22a) of the carrier substrate area (22),
- wherein the layer structure for the electrode (26) is formed with at least one or of at least one primary metal area (26-1) arranged bottom most, a subsequent auxiliary layer (26-2) as an alloy and diffusion barrier (26-2) and an actual electrode layer (26-3) arranged top most,
- wherein the primary metal area (26-1) is formed with or of copper,
- wherein the auxiliary layer (26-2) is formed with or of nickel,
- wherein the actual electrode layer (26-3) arranged uppermost is formed with or of a noble metal.

2. Sensor electrode arrangement according to claim 1,
**characterized in**
**that** the carrier substrate area (22) exhibits a chemically inert, biologically inert and electrically insulating material or is formed of such a material.

3. Sensor electrode arrangement according to one of the preceding claims,
**characterized in**
**that** the carrier substrate area (22) exhibits a mechanically flexible material or is formed as such, in particular in the form or the manner of a film.

4. Sensor electrode arrangement according to one of the preceding claims,
**characterized in**
**that** the primary metal area (26-1) is formed by being processed photolithographically, bonded on, laminated on and/or printed on.

5. Sensor electrode arrangement according to one of the preceding claims,
**characterized in**
**that** the auxiliary layer (26-2), the actual electrode layer (26-3) arranged uppermost or both are formed by electrodeposition.

6. Sensor electrode arrangement according to one of the preceding claims,
**characterized in**
**that** the carrier substrate area (22) is formed entirely or partly of a chemically inert, biologically inert material and a material at most slightly absorptive vis-à-vis proteins, biologically and chemically active principles.

7. Sensor electrode arrangement according to one of the preceding claims,
**characterized in**
**that** the carrier substrate area (22) is formed with or of a material from the group consisting of PMMA, PTFE, POM, FR4, polyimide, PI, Kapton, PEN, PET and materials transparent in the UV range.

8. Sensor electrode arrangement according to one of the preceding claims,
**characterized in**
**that** a plurality of identical intermediate substrate areas (26) and biomaterial areas (24) is formed in a form electrically insulated from each other and laterally arranged side by side on the carrier substrate area (22).

9. Sensor electrode arrangement according to claim 8,
**characterized in**
**that** the plurality of intermediate substrate areas (26) and biomaterial areas (24) are arranged in sequence or in matrix form.

10. Sensor electrode arrangement according to one of the preceding claims, which is formed as a sensor electrode arrangement for amperometric and/or potentiometric, pharmacological active site and/or active principle testing.

11. Sensor electrode arrangement according to any one of the preceding claims,
wherein a plurality of primary carriers (10) is provided adsorbed onto the biomaterial area (24), the primary carriers being activable to electronic action and biological, in particular membrane proteins.

12. Sensor electrode arrangement according to claim 18,
- wherein as a primary carrier (10), a primary carrier from the group is provided which consists of a eukaryotic cell, a prokaryotic cell, a bacterium, a virus, components, membrane fragments, or associations thereof in the native form or in a modified form, in particular in the purified form or a form modified microbiologically and by molecular biology or
- wherein as a primary carrier (10), a primary carrier of the group is provided which consists of a vesicle, a liposome or a micellar structure.

13. Sensor electrode arrangement according to one of the preceding claims,
**characterized in**
**that** area insulating and covering the electrode (26), of the biomaterial area (24) or the insulation area (24) is formed with a membrane structure (SSM) with a surface of approximately A ≈ 0.1 - 50 mm² and with a specific electric conductivity of approximately Gₘ ≈ 1 - 100 nS/cm² and/or with a specific capacitance of approximately Cₘ ≈ 10 - 1000 nF/cm²_{.}

14. Sensor electrode arrangement according to one of the preceding claims,
**characterized in**
**that** a biological unit (12) is provided which is formed activable to electrogenic charge carrier movement or to electrogenic charge carrier transportation.

15. Sensor electrode arrangement according to one of the preceding claims,
**characterized in**
**that** a membrane protein, an ion pump, an ion channel, a transporter, receptor, a component or an association thereof is provided as biological unit (12) in each case.

16. Sensor electrode arrangement according to one of the preceding claims,
**characterized in**
**that** the biological unit (12) is provided in the native form or in a modified, purified form, a form modified microbiologically or a form modified by molecular biology.

17. Sensor electrode arrangement according to one of the preceding claims,
**characterized in**
- **that** the surface (10a) of the primary carriers (10) and the surface of the secondary carrier (20) are formed with an opposite polarity or charge or
- **that**, between the surface (10a) of the primary carriers (10) and the surface of the secondary carrier (20) a connection of the type of a chemical bond is formed via a His-Tag coupling or a streptavidin biotin coupling.

18. Process for the manufacture of a biocompatible sensor electrode arrangement (20),
- wherein at least one carrier substrate area (22) is formed with a top side with a surface area (22a),
- wherein at least one electrical conductive electrode (26) is formed on the surface area (22a) of the carrier substrate area (22) or a part thereof with a top side facing away from the carrier substrate area (22) with a surface area (26a) and
- wherein a biomaterial area (24) is formed on the surface area (26a) of the electrode (26) or a part thereof with at least one biologically compatible material component,
- the biomaterial area (24) being provided as an electrically insulating insulation area (24),
- in operation, the electrode (26) concerned being electrically insulated by the biomaterial area (24) from a measuring medium (30), from the primary carriers (10) and from the biological units (12),
- the biomaterial area (24) being formed as layers,
- the biomaterial area (24) being formed at least partly of a sequence of monolayers (24a, 24b),
- the monolayers (24a,24b) being formed as spontaneously self-organising layers
- as sub-layer (24b) of the biomaterial area (24), a layer of a long-chain alkane thiol being provided as bottom most area (24b) facing towards the electrode (26) of the biomaterial area (24),
- as top layer (24a) of the biomaterial area (24), a layer of a lipid being provided as uppermost area facing away from the electrode (26) or surface area (24a) of the biomaterial area (24),
- the carrier substrate area (22) with the electrode (26) thereon as such or the electrode (26) as such on the one hand and the biomaterial area (24) on the other hand are being formed in a structured manner, namely in the form or in the manner of a wafer element or a printed circuit,
- wherein for the electrode (26), a metallic layer structure is formed on the surface (22a) of the carrier substrate area (22),
- wherein the layer structure for the electrode (26) is formed with at least one or of at least one primary metal area (26-1) arranged bottom most, a subsequent auxiliary layer (26-2) as alloy and diffusion barrier (26-2) and an actual electrode layer (26-3) arranged top most,
- wherein the primary metal area (26-1) is formed with or of copper,
- wherein the auxiliary layer (26-2) is formed with or of nickel, and
- wherein the actual electrode layer (26-3) arranged uppermost is formed with or of a noble metal.

19. Process according to claim 18,
wherein the carrier substrate (22) with the electrode (26) thereon or the electrode (26) as such or a part thereof in each case are formed:
- as a or with a photolithographically processed structure or as a or with a photographically processed element
- as a or with a structure being bonded on or laminated on or as an or with an element processed by being bonded on or laminated on,
- as a or with a structure processed micromechanically and/or by laser ablation or as an or with an element processed micromechanically and/or by laser ablation and/or
- as a or with a structure processed by printing or as an or with a element processed by printing,
in particular on the carrier substrate (22) in each case.

20. Process according to one of the preceding claims 18 or 19.
**characterized in**
**that** the carrier substrate area (22) is formed with a chemically inert, biologically inert and electrically insulating material or of such a material.

21. Process according to one of the preceding claims 18 to 20,
**characterized in**
**that** the carrier substrate area (22) is formed with a mechanically flexible material or of such a material, in particular in the form or manner of a film.

22. Process according to one of the preceding claims 18 to 21,
**characterized in**
**that** the primary metal area (26-1) is formed by being processed photolithographically, bonded on, laminated on or printed on.

23. Process according to one of the preceding claims 18 to 22,
**characterized in**
**that** the auxiliary layer (26-2), the actual electrode layer (26-3) arranged top most, or both are formed by electrodeposition.

24. Process according to one of the preceding claims 25 to 35,
**characterized in**
**that** the carrier substrate area (22) is formed entirely or partly of a chemically inert, biologically inert material and/or a material at most slightly absorptive vis-à-vis proteins, biologically and/or chemically active principles.

25. Process according to one of the preceding claims 18 to 24,
**characterized in**
**that** the carrier substrate area (22) is formed entirely or partially of PMMA, PTFE, POM, FR4, polyimide such as e.g. PI or Kapton, PEN, PET and/or of a material which is transparent - in particular in the UV range.

26. Process according to one of the preceding claims 18 to 25,
**characterized in**
**that** a plurality of identical electrodes (26) and biomaterial areas (24) is formed in a connected or in separated form, in particular in a form electrically insulated from each other.

27. Process according to claim 26,
**characterized in**
**that** the plurality of electrodes (26) and biomaterial areas (24) are arranged in sequence or in matrix form.

28. Process according to one of the preceding claims 18 to 27,
wherein the sensor electrode arrangement is formed as sensor electrode arrangement for amperometric and/or potentiometric, pharmacological active site and/or active principle testing with a counter electrode (46).

29. Process according to claims 18 to 28,
wherein a plurality of primary carriers (10) is provided adsorbed onto the biomaterial area (24), the primary carriers containing units (12) which are activable to electronic action and biological, in particular membrane proteins.

30. Process according to claim 29,
- in which, as primary carriers (10), a primary carrier from the group is provided in each case which exhibits a eukaryotic cell, a prokaryotic cell, a bacterium, a virus or components, membrane fragments, or associations thereof in the native form or in a modified, purified form or a form modified microbiologically or by molecular biology or
- in which, as primary carrier (10), a primary carrier from the group is provided in each case which exhibits a vesicle, a liposome or a micellar structure.

31. Process according to one of the preceding claims 18 to 30,
**characterized in**
**that** the area, insulating and covering the electrode (26), of the biomaterial area (24) or the insulation area (24) is formed with a membrane structure (SSM) with a surface of approximately A ≈ 0.1 - 50 mm² and with a specific electric conductivity of approximately Gₘ ≈ 1 - 100 nS/cm² and/or with a specific capacitance of approximately Cₘ ≈ 10 - 1000 nF/cm².

32. Process according to one of the preceding claims 18 to 31,
**characterized in**
**that** a biological unit (12) is provided which is formed activable to electrogenic charge carrier movement, in particular to electrogenic charge carrier transportation.

33. Process according to one of the preceding claims 18 to 32,
**characterized in**
**that** a membrane protein, in particular an ion pump, an ion channel, a transporter or receptor or a component or an association thereof is provided as biological unit (12) in each case.

34. Process according to one of the preceding claims 18 to 33,
**characterized in**
**that** the biological unit (12) is provided in the native form or in a modified form, in particular in a purified form, a form modified microbiologically and/or a form modified by molecular biology.

35. Process according to one of the preceding claims 18 to 34,
**characterized in**
- **that** the surface (10a) of the primary carriers (10) and the surface of the secondary carrier (20) are formed with an opposite polarity or charge and/or
- **that**, between the surface (10a) of the primary carriers (10) and the surface of the secondary carrier (20) a connection of the type of a chemical bond is formed, in particular via a His-Tag coupling or a streptavidin biotin coupling.

## Revendications

1. Dispositif à électrode de détection biocompatible (20)
- avec au moins une zone de substrat (22) dont la face supérieure comporte une zone superficielle (22a),
- avec au moins une électrode (26) conductrice d'électricité qui est formée sur la zone superficielle (22a) de la zone de substrat (22) ou sur une partie de celle-ci, et dont la face supérieure opposée à ladite zone de substrat (22) comporte une zone superficielle (26a), et
- avec une zone en biomatériau (24) qui est formée sur la zone superficielle (26a) de l'électrode (26) ou sur une partie de celle-ci et qui comporte au moins un composant de matériau biocompatible,
- étant précisé que la zone en biomatériau (24) est prévue comme zone isolante électriquement,
- que grâce à la zone en biomatériau (24), l'électrode (26) est isolée électriquement, en fonctionnement, par rapport à un agent de mesure (30), aux supports primaires (10) et aux unités biologiques (12),
- que la zone en biomatériau (14) a une structure stratifiée,
- que la zone en biomatériau (24) se compose au moins en partie d'une succession de monocouches (24a, 24b),
- que les monocouches (24a, 26b) sont conçues comme des couches à auto-organisation spontanée,
- qu'il est prévu comme couche inférieure (24b) de la zone en biomatériau (24) une couche d'alkanethiol à longue chaîne, sous la forme d'une zone inférieure (24b) de la zone en biomatériau (24) tournée vers l'électrode (26),
- qu'il est prévu comme couche supérieure (24a) de la zone en biomatériau (24) une couche composée d'un lipide, sous la forme d'une zone supérieure ou d'une zone superficielle de la zone en biomatériau (24) opposée à l'électrode (26),
- que la zone de substrat (22) avec l'électrode (26) sur celle-ci, ou l'électrode (26) d'une part et la zone en biomatériau (24) d'autre part sont formées de manière structurée, à savoir sous la forme ou à la manière d'un élément formant platine ou d'un circuit imprimé,
- que pour l'électrode (26), une structure stratifiée métallique est formée sur la surface (22a) de la zone de substrat (22),
- que la structure stratifiée pour l'électrode (26) comporte au moins une zone métallique primaire (26-1) disposée au-dessous, une couche auxiliaire suivante (26-2) comme barrière d'alliage et de diffusion, et une couche d'électrode proprement dite (26-3) disposée au-dessus,
- que la zone métallique primaire (26-1) comporte du cuivre ou se compose de cuivre,
- que la couche auxiliaire (26-2) comporte du nickel ou se compose de nickel, et
- que la couche d'électrode proprement dite (26-3) disposée au-dessus comporte un métal précieux ou se compose de métal précieux.

2. Dispositif à électrode de détection selon la revendication 1, **caractérisé en ce que** la zone de substrat (22) comporte un matériau chimiquement inerte et électriquement isolant ou se compose d'un tel matériau.

3. Dispositif à électrode de détection selon l'une des revendications précédentes, **caractérisé en ce que** la zone de substrat (22) comporte ou est conçue comme un matériau mécaniquement flexible, en particulier sous la forme ou à la manière d'un film.

4. Dispositif à électrode de détection selon l'une des revendications précédentes, **caractérisé en ce que** la zone métallique primaire (26-1) est formée par photolithographie, par collage, par laminage et/ou par impression.

5. Dispositif à électrode de détection selon l'une des revendications précédentes, **caractérisé en ce que** la couche auxiliaire (26-2), la couche d'électrode proprement dite (26-3) prévue au-dessus ou les deux sont formées par galvanisation.

6. Dispositif à électrode de détection selon l'une des revendications précédentes, **caractérisé en ce que** la zone de substrat (22) se compose entièrement ou partiellement d'un matériau chimiquement inerte et au maximum faiblement adsorbant par rapport aux protéines et aux substances actives biologiques et chimiques.

7. Dispositif à électrode de détection selon l'une des revendications précédentes, **caractérisé en ce que** la zone de substrat (22) comporte un matériau choisi dans le groupe constitué par PMMA, PTFE, POM, FR4, polyimide, PI, Kapton, PEN, PET et par des matériaux transparents dans la zone UV, ou se compose d'un tel matériau.

8. Dispositif à électrode de détection selon l'une des revendications précédentes, **caractérisé en ce que** plusieurs électrodes (26) et zones en biomatériau (24) de même type sont formées sous une forme isolée électriquement et en étant juxtaposées latéralement sur la zone de substrat (22).

9. Dispositif à électrode de détection selon la revendication 8, **caractérisé en ce que** les électrodes (26) et les zones en biomatériau (24) sont disposées sur une rangée ou suivant une forme de matrice.

10. Dispositif à électrode de détection selon l'une des revendications précédentes, qui comporte une contre-électrode (46) pour un test ampérométrique et/ou potentiométrique, pharmacologique de lieu actif et/ou de substance active.

11. Dispositif à électrode de détection selon l'une des revendications précédentes, avec lequel plusieurs supports primaires (10) sont prévus en étant adsorbés sur la zone en biomatériau (24), les supports primaires contenant en vue d'une action électrique des unités activables et biologiques (12), en particulier des protéines membranaires.

12. Dispositif à électrode de détection selon la revendication 11,
- dans lequel il est prévu comme support primaire (10) un support primaire du groupe qui est constitué par une cellule eucaryotique, une cellule procaryotique, une bactérie, un virus, des composants, des fragments de membrane ou des groupements de ceux-ci, sous une forme native ou sous une forme modifiée, en particulier sous une forme purifiée, modifiée d'un point de vue de la microbiologie, de la biologie moléculaire, et
- dans lequel il est prévu comme support primaire (10) un support primaire du groupe qui est constitué par une vésicule, un liposome et une structure micellaire.

13. Dispositif à électrode de détection selon l'une des revendications précédentes, **caractérisé en ce que** la partie de la zone en biomatériau (24) ou de la zone isolante (24) qui isole et couvre l'électrode (26) présente une structure de membrane (SSM) d'une surface d'environ A≈0,1-50 mm² et une conductivité électrique spécifique d'environ Gₘ≈1-100 S/cm² et/ou une capacité spécifique d'environ Cₘ≈10-1000 nF/cm².

14. Dispositif à électrode de détection selon l'une des revendications précédentes, **caractérisé en ce qu'**il est prévu une unité biologique (12) qui est activable en vue d'un mouvement de support de charge électrogène ou d'un transport de support de charge électrogène.

15. Dispositif à électrode de détection selon l'une des revendications précédentes, **caractérisé en ce qu'**il est prévu comme unité biologique (12) une protéine membranaire, une pompe ionique, un canal ionique, un transporteur, un récepteur, un composant ou un groupement de ceux-ci.

16. Dispositif à électrode de détection selon l'une des revendications précédentes, **caractérisé en ce que** l'unité biologique (12) est prévue sous une forme native ou sous une forme modifiée, purifiée, modifiée d'un point de vue de la microbiologie ou de la biologie moléculaire.

17. Dispositif à électrode de détection selon l'une des revendications précédentes, **caractérisé**
- **en ce que** la surface (10a) des supports primaires (10) et la surface de la zone en biomatériau (24) sont conçues avec des polarités ou des charges opposées, ou
- **en ce qu'**il est prévu entre la surface (10a) des supports (10) et la surface de la zone en biomatériau (24) un couplage à la manière d'une liaison chimique, par l'intermédiaire d'un couplage His-Tag ou d'un couplage streptavidine-biotine.

18. Procédé pour fabriquer un dispositif à électrode de détection biocompatible (20),
- selon lequel on dote au moins une zone de substrat (22) d'une face supérieure avec une zone superficielle (22a),
- selon lequel on dote au moins une électrode (26) conductrice d'électricité qui est formée sur la zone superficielle (22a) de la zone de substrat (22) ou sur une partie de celle-ci d'une face supérieure opposée à ladite zone de substrat (22) et comportant une zone superficielle (26a), et
- selon lequel on dote une zone en biomatériau (24) qui est formée sur la zone superficielle (26a) de l'électrode (26) ou sur une partie de celle-ci d'au moins un composant de matériau biocompatible,
- étant précisé que la zone en biomatériau (24) est prévue comme zone isolante électriquement (24),
- que grâce à la zone en biomatériau (24), l'électrode (26) est isolée électriquement, en fonctionnement, par rapport à un agent de mesure (30), aux supports primaires (10) et aux unités biologiques (12),
- que la zone en biomatériau (14) a une structure stratifiée,
- que la zone en biomatériau (24) se compose au moins en partie d'une succession de monocouches (24a, 24b),
- que les monocouches (24a, 26b) sont conçues comme des couches à auto-organisation spontanée,
- qu'on prévoit comme couche inférieure (24b) de la zone en biomatériau (24) une couche d'alkanethiol à longue chaîne, comme zone inférieure (24b) de la zone en biomatériau (24), tournée vers l'électrode (26),
- qu'il est prévu comme couche supérieure (24a) de la zone en biomatériau (24) une couche composée d'un lipide, comme zone supérieure et opposée à l'électrode (26), ou comme zone superficielle (24a) de la zone en biomatériau (24),
- que la zone de substrat (22) avec l'électrode (26) sur celle-ci, ou l'électrode (26) d'une part et la zone en biomatériau (24) d'autre part sont formées de manière structurée, à savoir sous la forme ou à la manière d'un élément formant platine ou d'un circuit imprimé,
- que pour l'électrode (26), on forme une structure stratifiée métallique sur la surface supérieure (22a) de la zone de substrat (22),
- que la structure stratifiée pour l'électrode (26) comporte ou se compose d'au moins une zone métallique primaire (26-1) disposée au-dessous, d'une couche auxiliaire suivante (26-2) comme barrière d'alliage et/ou de diffusion (26-2), et d'une couche d'électrode proprement dite (26-3) disposée au-dessus,
- que la zone métallique primaire (26-1) comporte du cuivre ou se compose de cuivre,
- que la couche auxiliaire (26-2) comporte du nickel ou se compose de nickel, et
- que la couche d'électrode proprement dite (26-3) disposée au-dessus comporte un métal précieux ou se compose de métal précieux.

19. Procédé selon la revendication 18, selon lequel on conçoit la zone de substrat (22) avec l'électrode (26) sur celle-ci, ou l'électrode (26) en soi ou une partie de celle-ci :
- comme ou avec une structure traitée par photolithographie, ou comme ou avec un élément traité par photolighographie,
- comme ou avec une structure traitée par collage ou laminage, ou comme ou avec un élément traité par collage ou laminage,
- comme ou avec une structure traitée micromécaniquement et/ou par ablation au laser, ou comme ou avec un élément traité micromécaniquement et/ou par ablation au laser et/ou
- comme ou avec une structure traitée par impression, ou comme ou avec un élément traité par impression.

20. Procédé selon la revendication 18 ou 19, **caractérisé en ce qu'**on forme la zone de substrat (22) avec un matériau chimiquement inerte ou électriquement isolant, ou à partir d'un tel matériau.

21. Procédé selon l'une des revendications 18 à 20 précédentes, **caractérisé en ce qu'**on forme la zone de substrat (22) avec un matériau mécaniquement flexible ou à partir d'un tel matériau, en particulier sous la forme ou à la manière d'un film.

22. Procédé selon l'une des revendications 18 à 21 précédentes, **caractérisé en ce qu'**on forme la zone métallique primaire (26-1) par photolithographie, par collage, par laminage ou par impression.

23. Procédé selon l'une des revendications 18 à 22 précédentes, **caractérisé en ce qu'**on forme la couche auxiliaire (26-2), la couche d'électrode proprement dite (26-3) prévue au-dessus ou les deux par galvanisation.

24. Procédé selon l'une des revendications 18 à 23 précédentes, **caractérisé en ce qu'**on forme la zone de substrat (22) entièrement ou partiellement à partir d'un matériau chimiquement inerte et/ou au maximum faiblement adsorbant par rapport aux protéines et aux substances actives biologiques et/ou chimiques.

25. Procédé selon l'une des revendications 18 à 24 précédentes, **caractérisé en ce qu'**on forme la zone de substrat (22) entièrement ou partiellement à partir de PMMA, PTFE, POM, FR4, polyimide, comme par exemple PI ou Kapton, PEN, PET et/ou à partir d'un matériau transparent - en particulier dans la zone UV.

26. Procédé selon l'une des revendications 18 à 25 précédentes, **caractérisé en ce qu'**on forme plusieurs électrodes (26) et zones en biomatériau (24) de même type d'un seul tenant ou séparément, sous une forme isolée électriquement.

27. Procédé selon la revendication 26, **caractérisé en ce qu'**on dispose les électrodes (26) et les zones en biomatériau (24) sur une rangée ou suivant une forme de matrice.

28. Procédé selon l'une des revendications 18 à 27 précédentes, selon lequel on dote le dispositif à électrode de détection d'une contre-électrode (46) pour un test ampérométrique et/ou potentiométrique, pharmacologique de lieu actif et/ou de substance active.

29. Procédé selon l'une des revendications 18 à 28 précédentes, selon lequel on prévoit plusieurs supports primaires (10) adsorbés sur la zone en biomatériau (24), les supports primaires contenant en vue d'une action électrique des unités activables et biologiques (12), en particulier des protéines membranaires.

30. Procédé selon la revendication 29,
- selon lequel on prévoit comme support primaire (10) un support primaire du groupe qui est constitué par une cellule eucaryotique, une cellule procaryotique, une bactérie, un virus ou des composants, des fragments de membrane ou des groupements de ceux-ci, sous une forme native ou sous une forme modifiée, purifiée, modifiée d'un point de vue de la microbiologie, de la biologie moléculaire, ou
- selon lequel on prévoit comme support primaire (10) un support primaire du groupe qui est constitué par une vésicule, un liposome et une structure micellaire.

31. Procédé selon l'une des revendications 18 à 30 précédentes, **caractérisé en ce qu'**on dote la zone de la zone en biomatériau (24) qui isole et couvre l'électrode (26) d'une structure de membrane (SSM) avec une surface d'environ A≈0,1-50 mm² et d'une conductivité électrique spécifique d'environ Gₘ≈1-100 nS/cm² et/ou d'une capacité spécifique d'environ Cₘ≈10-1000 nF/cm².

32. Procédé selon l'une des revendications 18 à 31 précédentes, **caractérisé en ce qu'**on prévoit une unité biologique (12) qui est activable en vue d'un mouvement de support de charge électrogène, en particulier en vue d'un transport de support de charge électrogène.

33. Procédé selon l'une des revendications 18 à 32 précédentes, **caractérisé en ce qu'**on prévoit comme unité biologique (12) une protéine membranaire, en particulier une pompe ionique, un canal ionique, un transporteur ou un récepteur ou un composant ou un groupement de ceux-ci.

34. Procédé selon l'une des revendications 18 à 33 précédentes, **caractérisé en ce qu'**on prévoit l'unité biologique (12) sous une forme native ou sous une forme modifiée, en particulier purifiée, modifiée d'un point de vue de la microbiologie et/ou de la biologie moléculaire.

35. Procédé selon l'une des revendications 18 à 34 précédentes, **caractérisé**
- **en ce que** la surface (10a) des supports primaires (10) et la surface de la zone en biomatériau (24) sont conçues avec des polarités ou des charges opposées, et/ou
- **en ce qu'**on prévoit entre la surface (10a) des supports (10) et la surface de la zone en biomatériau (24) un couplage à la manière d'une liaison chimique, par l'intermédiaire d'un couplage His-Tag ou d'un couplage streptavidine-biotine.
